# EUROPEAN PATENT SPECIFICATION

(11) **EP 2 523 946 B1**
(45) Date of publication and mention of the grant of the patent: **20.09.2017**
(21) Application number: 11700612.2
(22) Date of filing: 13.01.2011
(51) Int. Cl.: C07D 307/93, A61K 8/49, A61K 8/97, A61K 31/343, A61K 31/365, A61P 3/04, A61P 3/06, A61Q 19/06

(54) **Tricyclic sesquiterpene lactones for used in the treatment of obesity and related diseases and non-therapeutic treatable conditions**
Trizyklische Sesquiterpen-Lactone zur Behandlung von Fettleibigkeit und zugehörigen Störungen sowie von nichttherapeutisch behandelbaren Zuständen
Lactones de sesquiterpène tricyclique dans le traitement de l'obésité, de troubles associés et de conditions traitables non thérapeutiques

(30) Priority: 14.01.2010 EP 10000282
(43) Date of publication of application: 21.11.2012
(60) Divisional application: 17185407.8
(73) Proprietor: Neem Biotech Ltd., Abertillery, Blaenau Gwent NP13 1SX (GB)
(72) Inventor: GROTHE, Torsten, 44809 Bochum (DE); ROEMER, Ernst, 07751 Bucha (DE); WABNITZ, Philipp, 40237 Düsseldorf (DE)
(74) Representative: Johnston, Magnus George
(86) International application number: PCT/EP2011/000110
(87) International publication number: WO 2011/085979

(56) References cited:
- WO-A1-03/015765
- WO-A1-2004/096252
- WO-A1-2009/005115
- WO-A2-2007/006391
- KR-A- 20040 070 985
- KR-A- 20040 071 336
- SHIMODA, HIROSHI ET AL: "Anti-Hyperlipidemic sesquiterpenes and new sesquiterpene glycosides from the leaves of artichoke (Cynara scolymus L.): structure requirement and mode of action", BIOORGANIC & MEDICINAL CHEMISTRY LETTERS, vol. 13, no. 2, 2003, pages 223-228, XP002601824,
- FRITSCHE, JAN ET AL: "Isolation, characterization and determination of minor artichoke (Cynara scolymus L.) leaf extract compounds", EUROPEAN FOOD RESEARCH AND TECHNOLOGY, vol. 215, no. 2, 2002, pages 149-157, XP002601825,
- DATABASE CA [Online] CHEMICAL ABSTRACTS SERVICE, COLUMBUS, OHIO, US; 2006, YOSHIKAWA, MASAYUKI: "Search for biofunctional molecules from medicinal foods", XP002602056, retrieved from STN Database accession no. 2006:1150177
- DATABASE CA [Online] CHEMICAL ABSTRACTS SERVICE, COLUMBUS, OHIO, US; 2007, AHN, EUN-MI ET AL: "Cytotoxic and ACAT-inhibitory sesquiterpene lactones from the root of Ixeris dentata forma albiflora", XP002602057, retrieved from STN Database accession no. 2007:907
- GONZALEZ COLLADO, I. ET AL: "Structure and chemistry of secondary metabolites from Compositae. Part 4. Guaianolides fro Centaurea canariensis", PHYTOCHEMISTRY, vol. 24, no. 9, 1985, pages 2107-2109, XP002601826,
- GONZALEZ, ANTONIO G. ET AL: "Constituents of the Compositae. Part 42. Subexpinnatin, a new guaianolide from Centaurea canariensis", PHYTOCHEMISTRY, vol. 21, no. 4, 1982, pages 895-897, XP002601827,
- CRAVOTTO, GIANCARLO ET AL: "Chemical and biological modification of cynaropicrin and grosheimin: A structure-bitterness relationship study", JOURNAL OF THE SCIENCE OF FOOD AND AGRICULTURE, vol. 85, no. 10, 2005, pages 1757-1764, XP002601828,
- SAMEK, Z. ET AL: "Terpenes. CCXIII. Sesquiterpenic lactones of the Cynara scolymus species", TETRAHEDRON LETTERS, vol. 50, 1971, pages 4775-4778, XP002601829,
- LI, XIAOLI ET AL: "Sesquiterpenoids from Cynara scolymus", HETEROCYCLES, vol. 65, no. 2, 2005, pages 287-291, XP008127188,
- OMAR, ABDALLAH A. ET AL: "Guaianolides from Cynara sibthorpiana", PHYTOCHEMISTRY, vol. 23, no. 10, 1984, pages 2381-2382, XP002601830,
- MASSANET, G. M. ET AL: "Structure and chemistry of secondary metabolites from Compositae. Part 1. Integrifolin , a guaianolide from Andryala integrifolia", PHYTOCHEMISTRY, vol. 23, no. 4, 1984, pages 912-913, XP002601831,
- GEBHARD, ROLF: "Inhibition of cholesterol biosynthesis in primary cultured rat hepatocytes by artichoke (Cynara scolymus L.) extracts", JOURNAL OF PHARMACOLOGY AND EXPERIMENTAL THERAPEUTICS, vol. 286, no. 3, 1998, pages 1122-1128, XP002601836,
- WANG, YING ET AL: "Neurotoxic sesquiterpenoids from the yellow star thistle Centaurea solstitialis I. (Asteraceae)", HELVETICA CHIMICA ACTA, vol. 74, no. 1, 1991, pages 117-123, XP002601833,

## Description

### Summary

The disclosure relates to the use, or methods (especially with regard to animals, especially human, that are in need of such treatment) comprising the use, of an extract and/or especially one or more natural compounds from plants or parts of plants comprising tricyclic sesquiterpene lactones, especially Cynaropicrin, respectively, from a genus as defined below of alone or as supplement, as active ingredient in the regulation of body weight and/or fat loss and/or for the management of obesity; and/or for improving the total cholesterol HDULDL ratio; either in humans or in other warm-blooded animals, and/or to the use of said extract and/or natural compound(s) or mixtures in the manufacture of a pharmaceutical or nutraceutical formulation for the regulation of body weight and/or fat loss and/or for the management of obesity; and/or for improving the total cholesterol HDULDL ratio; either in humans or other warm-blooded animals. The disclosure relates also to said extract and/or compound(s) for use in the treatment or in the preparation of a medicament (including a nutraceutical) for the treatment of obesity; and/or for improving the total cholesterol HDULDL ratio; as well as their preparation. It also relates to pharmaceutical or nutraceutical formulations comprising said extract and/or natural compound(s) which are useful in the regulation of body weight and/or fat loss and/or for the management of obesity; and/or for improving the total cholesterol HDULDL ratio. They can also be used for non-therapeutic, e.g. cosmetic, purposes only.

### Background of the invention

Weight control is an important concern of human beings, both for medical (pharmaceutical and/or nutraceutical) as well as non-therapeutic, e.g. cosmetic, reasons. More importantly, excessive accumulation of body fat (i.e. obesity (= adiposity), especially with excessive fat in the ventral region and surrounding the viscera) can be dangerous and has been linked to health problems such as type II diabetes, hypertension, heart disease, atherosclerosis (where more than two of the preceding disorders are present, the condition is often called "Metabolic Syndrome" or "syndrome X"), hyperlipidemia, coronary heart disease, stroke, breast and colon cancer, sleep apnoea, gallbladder disease, reproductive disorders such as polycystic ovarian syndrome, gastroesophageal reflux disease, increased incidence of complications of general anesthesia, fatty liver, gout or thromboembolism (see, e.g., Kopelman, Nature 404: 635-43 (2000)). Obesity reduces life-span and carries a serious risk of the co-morbidities listed above, as well disorders such as infections, varicose veins, acanthosis nigricans, eczema, exercise intolerance, insulin resistance, hypertension hypercholesterolemia, cholelithiasis, orthopedic injury, and thromboembolic disease (Rissanen et al, Br. Med. J. 301: 835-7 (1990)). Obesity is one of the main factors in the development of cardiovascular diseases. As a side effect the levels of cholesterol, blood pressure, blood sugar and uric acid in obese people are usually higher than those of persons of normal weight. The morbidity from coronary heart disease among the overweight people is increased as well. Among the people aged 40-50, mortality will rise about 1% when body weight increases by 0.5 kg and the death rate will increase 74% when body weight exceeds 25% of the standard. The prevalence of obesity in the United States has more than doubled since the turn of the last century (whole population) and more than tripled within the last 30 years among children aged from 6 to 11. This problem more and more becomes a disease risk also in Europe. In Germany, particularly many people have been found to suffer from overweight recently, already 25% of the young people, children and adolescents there are affected by obesity and related disorders. Furthermore, being overweight is considered by the majority of the Western population as unattractive.

Overweight and obesity result from an imbalance between the calories consumed and the calories used by the body. When the calories consumed exceed the calories burned, the body is in positive energy balance and over time weight gain will occur. The excess calories are stored in the fat cells. When the calories burned exceed the calories consumed, the body is in negative energy balance and over time weight loss will occur.

Determinants of obesity include social factors, psychological factors, genetic factors, developmental factors and decreased physical activity. Some components of a comprehensive weight loss programs include medical assessment, behavioural and dietary modification, nutrition education, mental and cognitive restructuring, increased physical activity, and long term follow-up.

An increasing interest by consumers in the maintenance or reduction of their body weight can be found. This leads to a demand for products useful for these purposes. Preferred are such food products which can conveniently be consumed as part of the daily diet, for example meal replacer products, such as meal replacer bars and beverages. These are usually designed for use as a single-serving food product to replace one or two meals a day.

An issue is that often a saturating effect is missed when such products are consumed, resulting in hunger feelings only a relatively short time after consummation or even in the lack of a saturation feeling already directly after consummation.

Summing up, there remains a need for new safe and effective compositions for promoting weight loss and/or loss of body fat in subjects such as humans. The problem to be solved by the present disclosure is therefore to find compositions or compounds useful in the treatment of obesity; and/or for improving the total cholesterol HDULDL ratio.

Phytochemistry provides a large pool of compounds and compositions to be looked at whether they are able to solve this problem.

The present disclosure provides methods and compositions useful in the control, treatment and prevention of obesity and obesity-related conditions, disorders, and diseases; and/or and/or for improving the total cholesterol HDULDL ratio.

Rosinski, G., et al., Endocrinological Frontiers in Phyiological Insect Ecology, Wroclow Technical University Press, Wroclow 1989, describe that certain tricyclic sequiterpene lactones, such as grossheimin and repin, showed inhibition of larval growth and antifeeding activity in Mealworm (*Tenebrio molitor*)*.* Grossheimin shows no anti-feeding but little decrease of absorption of digested food constituents and a little decrease in efficiency in digesting. Repin exhibit low effects at all. Both compounds show no effect on lipid levels in blood.

Shimoda, H., et al, Bioinorganic & Medicinal Chemistry Letters 13 (2003), 223-228, describe that methanolic extracts from Artichoke (*Cynara sclolymus L.*) with cynaropicrin, aguerin B and grossheimin as components and certain sesquiterpene glycosides suppress serum triglyceride elevation in olive oil-loaded mice. Some of these compounds exhibit a moderate short term (2 hours after olive oil administration) anti-hyperlipidemic activity presented as a lowering of the serum triglyceride (serum TG) concentrations, the long term (6 hours) show in the case of cynaropicrin and aguerine B an increase of the serum TG. Furthermore the authors present data of the gastric emptying (GE) of a methanolic ectract of artichoke. They determine a significantly inhibited GE. However, as shown below, this mechanism is not an explanation for the anti obesity effect shown in the present disclosure (see Example 1). Fritzsche, J., et al., Eur. Food Res. Technol. 215, 149-157 (2002) describe the effect of certain isolated artichoke leaflet extract components with cholesterol lowering potential. Ahn, E.M-., et al, Arch Pharm. res. 29(11), 937-941, 2006, shows ACAT inhibitory activity for two sesquiterpene lactones. KR 20040070985 also shows an effect of certain sesquiterpene lactone derivatives on cholesterol biosynthesis involved enzymes. Gebhard, R., Phytother. Res. 16, 368-372 (2002) and J. Pharmacol. Exp. Ther. 286(3), 1122-1128 (1998), shows enforcement of cholesterol biosynthesis inhibition in HepG2 cells by artichoke extracts. WO 2007/006391 also claims reduction in cholesterol by certain *Cynara scolymus* variety extracts.

Other reported activities of tricyclic sesquiterpene lactones are antioxidant activity (European Food Research & Technology {2002), 215{2): 149-157), inhibitors of NF kb (Food Style 21 (2007), 11(6): 54-56; JP 2006-206532), serum triglyceride increase-inhibitory effect (Kagaku Kogyo (2006), 57(10): 740-745), hypoglycaemic effect (J. Trad. Med. (2003), 20(2): 57-61), bitter taste (DE 2654184).

Yoshikawa Masayuki: "Search for biofunctional molecules from medicinal foods", 2006, retrieved from STN Database accession no. 2006:1150177 relates to anti-obesity and anti-allergy components derived from plants, including Cynara scolymus-derived cynaropicrin having serum triglyceride increase-inhibitory effect. WO 03/015765 relates to sesquiterprenoid derivatives having adipocyte differentiation inhibitory effect. WO 2004/096252 relates to dietary supplements for management and control of body weight comprising at least one liver protecting agent and at least one mushroom in the form of powder, extract, or combinations thereof.

None of the documents suggest that a control and treatment of obesity and body fat in warm-blooded animals might be possible.

### General Description

The present invention is defined in and by the appended claims.
Surprisingly we have found that Cynaropicrin, a tricyclic sesquiterpene lactone causes *in vivo* a strong weight loss. More surprisingly it was found that this effect is not correlated to a decrease in food intake. The weight balance is not affected by reduction of assimilation efficiency; the decrease of body fat and body weight is presumably caused by effects on energy metabolism. Surprisingly, it was found in addition that cynaropicrin also allows for improving the total cholesterol HDL/LDL ratio

Tricyclic sequiterpene lactones or known ingredients of plants of the subclass *Asterides,* especially from the family of *Asteraceae,* more specifically from species of the genera of the list consisting of *Achilea, Acroptilon, Agranthus, Ainsliaea, Ajania, Amberboa, Andryala, Artemisia, Aster, Bisphopanthus, Brachylaena, Calea, Calycocorsus, Cartolepsis, Centaurea, Cheirolophus, Chrysanthemum, Cousinia, Crepis, Cynara, Eupatorium, Greenmaniella, Grossheimia, Hemistaptia, Ixeris, Jurinea, Lapsana, Lasiolaena, Liatris, Lychnophora, Macroclinidium, Mikania, Otanthus, Pleiotaxis, Prenanthes, Pseudostifftia, Ptilostemon, Rhaponticum, Santolina, Saussurea, Serratula, Sonchus, Stevia, Taeckholmia, Tanacetum, Tricholepis, Vernonia, Volutarella, Zaluzania*; even more specifically from species of the list consisting of *Achillea clypeolata, Achillea collina, Acroptilon repens, Agrianthus pungens, Ainsliaea fragrans, Ajania fastigiata, Ajania fruticulosa, Amberboa lippi, Amberboa muricata, Amberboa ramose**, Amberboa tubuliflora* and other *Amberboa spp.**, *Andryala integrifolia, Andryala pinnatifida, Artemisia absinthium, Artemisia cana, Artemisia douglasiana, Artemisia fastigiata, Artemisia franserioides, Artemisia montana, Artemisia sylvatica, Artemisia* *tripartita, Aster auriculatus, Bishopanthus soliceps, Brachylaena nereifolia, Brachylaena perrieri, Calea jamaicensis, Calea solidaginea, Calycocorsus stipitatus, Cartolepsis intermedia, Centaurea babylonica, Centaurea bella, Centaurea canariensis*, Centaurea clementei, Centaurea conicum, Centaurea dealbata, Centaurea declinata, Centaurea glastifolia, Centaurea hermanii, Centaurea hyrcanica, Centaurea intermedia, Centaurea janeri, Centaurea kalscyi, Centaurea kandavanensis, Centaurea kotschyi, Centaurea linifolia, Centaurea macrocephala, Centaurea musimomum, Centaurea nicolai, Centaurea pabotii, Centaurea pseudosinaica, Centaurea repens, Centaurea salonitana, Centaurea scoparia, Centaurea sinaica, Centaurea solstitialis, Centaurea tweediei* and other *Centaurea spp.* *, *Cheirolophus uliginosus, Chrysanthemum boreale, Cousinia canescens, Cousinia conifera, Cousinia picheriana, Cousinia piptocephala, Crepis capillaris, Crepis conyzifolia, Crepis crocea, Crepis japonica, Crepis pyrenaica, Crepis tectorum, Crepis virens, Crepis zacintha, Cynara alba, Cynara algarbiensis, Cynara auranitica, Cynara baetica, Cynara cardunculus, Cynara cornigera, Cynara cyrenaica, Cynara humilis, Cynara hystrix, Cynara syriaca, Cynara scolymus**, Cynara sibthorpiana* and other *Cynara spp.**, *Eupatorium anomalum, Eupatorium chinense, Eupatorium lindleyanum, Eupatorium mohrii, Eupatorium rotundifolium, Eupatorium semialatum, Greenmaniella resinosa, Grossheimia macrocephala*** and other *Grossheimia spp.**, *Hemisteptia lyrata, Ixeris chinensis, Ixeris debilis, Ixeris dentata, Ixeris repens, Ixeris stolonifera, Jurinea carduiformis, Jurinea derderioides, Jurinea maxima, Lapsana capillaris, Lapsana communis, Lasiolaena morii, Lasiolaena santosii, Liatris chapmanii, Liatris gracilis, Liatris pycnostachya, Lychnophora blanchetii, Macroclinidium trilobum, Mikania hoehnei, Otanthus maritimus, Pleiotaxis rugosa, Prenanthes acerifolia, Pseudostifftia kingii, Ptilostemon diacanthus, Ptilostemon gnaphaloides, Rhaponticum serratuloides, Santolina jamaicensis, Saussurea affinis, Saussurea elegans, Saussurea involucrata, Saussurea laniceps, Saussurea neopulchella*** and other *Sauusurea spp.**, *Serratula strangulata, Sonchus arborea, Stevia sanguinea, Taeckholmia arborea, Taeckholmia pinnata, Tanacetum fruticulosum, Tanacetum parthenium, Tricholepis glaberrima*** and other *Tricholepsis spp.*, Vernonia arkansana, Vernonia nitidula, Vernonia noveboracensis, Vernonia profuga, Vernonia sublutea, Volutarella divaricata, Zaluzania resinosa;* and can potentially be isolated from any part of the plants. Those genera and/or species marked with an asterisk (*) and especially those species marked with two asterisks (**) are especially preferred.

Appropriate plant material can be obtained from various sources, e.g. from:
Alfred Galke GmbH, Gittelde/Harz, Germany; Muggenburg Pflanzliche Rohstoffe, Bad Bramstedt, Germany; Friedrich Nature Discovery, Euskirchen, Germany; VitaPlant AG, Uttwil, Switzerland; Amorós Nature SL, Hostalric, Spain.

### Detailed description

In one embodiment, the disclosure relates to a compound of the formula I, 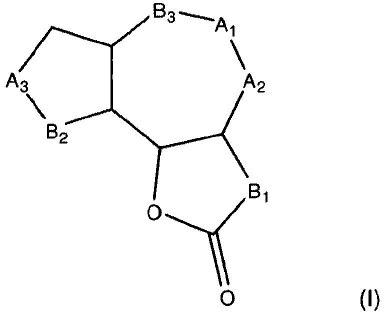 wherein
A1, A2 and A3 independently from each other represent the diradicals **CH₂, **CH-O-R1, or **C=O,
   with R1 denoting hydrogen, a straight-chain or branched-chain alkyl group having 1 to 12 carbon atoms, a cycloalkyl group having 3 to 10 carbon atoms and optionally comprising one or more ring heteroatoms, a carbohydrate having 5 to 12 carbon atoms, a substituted or unsubstituted aryl group with 6 to 12 ring atoms which can comprise one or more ring heteroatoms, a substituted or unsubstituted C₆-C₁₂-aryl-C₁-C₆-alkyl group which can comprise one or more heteroatoms, a substituted or unsubstituted aryloxy group wherein aryl has 6 to 12 ring atoms and can comprise one or more ring heteroatoms, a (-C(O)-Rₐ) group, a (-C(S)-Rₐ) group, a (-C(O)-ORₐ) group, a (-(CH₂)ₙ-CRₐ=CRₐR_{b}) group, or a (-C(O)-(CH₂)ₙ-CRₐ=CRₐR_{b}) group wherein n is zero or an integer from 1 to 12 and
   Rₐ and R_{b} independently from each other are selected from a group Z, consisting of hydrogen, a straight-chain or branched-chain alkyl group having 1 to 6 carbon atoms that is unsubstituted or substituted by hydroxy, a cycloalkyl group having 3 to 10 carbon atoms and optionally comprising one or more ring heteroatoms, and a substituted or unsubstituted aryl group with 6 to 12 ring atoms which can comprise one or more ring heteroatoms, and a substituted or unsubstituted C₆-C₁₂-aryl-C₁-C₆-alkyl group which can comprise one or more heteroatoms,
   and
B1, B2 and B3 independently from each other represent the diradicals **C=CH₂, **CH-CH₂-R1*, **COH-CH₂-X with X selected from the group F, Cl, and Br, **COH-CH₂-O-R1 with R1 as defined above and R1* being R1 as defined above or a straight-chain or branched-chain alkoxy group having 1 to 12 carbon atoms, or **C(Rx)(Ry) where Rx, Ry and the binding carbon atom together form a a cycloalkyl group having 3 to 10 carbon atoms and optionally comprising one or more ring heteroatoms,
where if one or more heteroatoms mentioned above are present they are present instead of one or more carbon atoms and are selected from the group consisting of S, N, NH, O, P and Se, preferably S, N, NH and O,
or a mixture of two or more compounds of the formula I, for use as active ingredient in the therapeutic - including prophylactic - treatment of a warm-blooded animal for the regulation of body weight (preferred) and/or fat loss (preferred) and/or for the management of obesity; and/or for improving the total cholesterol HDULDL ratio; where the compound(s) of the formula I may be present in free form, in the form of a pharmaceutically and/or nutraceutically acceptable salt, in the form of tautomers, in the form of esters and/or in the form of solvates, or alternatively or in addition in the form of an extract of the plant or plant parts of a plant of a genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella, especially a plant or plant parts of a plant of the species *Cynara,* more especially from *Cynara scolymus* or parts thereof, each comprising one or more compounds of the formula I in free form, in the form of a pharmaceutically and/or nutraceutically acceptable salt, in the form of tautomers, in the form of esters and/or in the form of solvates.

In all embodiments, either the essentially pure compound of formula I or mixtures of compounds of the formula I, or the compounds in the form of extracts comprising one or more such compounds, form separate embodiments , where the compound(s) of formula I in each case can be present in free form, in the form of a pharmaceutically and/or nutraceutically acceptable salt, in the form of tautomers, in the form of esters and/or in the form of solvates.

Further Embodiments of the disclosure relate to those mentioned under the definition of USE below.

In addition, also non-therapeutic use is possible, e.g. the USE of a compound of the formula I, or a mixture of two or more such compounds, for the cosmetic treatment of a warm-blooded animal, especially a human, comprising administering said compound or compound mixture to said animal, especially a human, in order to achieve cosmetically advantageous results; where the compound(s) can also be used in the form of a cosmetically acceptable (corresponding especially to pharmaceutically and/or nutraceutically acceptable as defined below) salt, in the form of tautomers, in the form of esters and/or in the form of solvates.

The general expressions, within the present disclosure, preferably have the following meaning, where in each embodiment one, more than one or all more general expressions may, independently of each other, be replaced with the more specific definitions, thus forming preferred embodiments of the disclosure, respectively:
Preferably, the compounds of the formula I are natural compounds, that is, compounds that are present in and can be isolated or extracted from natural sources (especially those mentioned in detail) without chemical synthesis steps (though they may also be prepared by chemical synthesis) and are thus present as extracts or purified components of extracts, and not derivatives only obtainable by chemical synthesis.

They can also be part of an extract which is obtainable by extracting a plant or a plant part from a plant of the family of Asteracea, especially from the genera mentioned above, especially a plant or plant parts of a plant of the species *Cynara,* more especially from *Cynara scolymus* or parts thereof.

Further, the present tricyclic sesquiterpene lactone derivatives of the formula I comprise all stereoisomers, such as those which may exist due to asymmetric carbons on the various substituents, including enantiomeric forms (which may exist even in the absence of asymmetric carbons, especially atropisomeres with respect to the different possible more or lass stable ring conformations of the cyclooctadiene moiety) and diastereomeric forms. Individual stereoisomers of the tricyclic sesquiterpene lactone derivatives of the present disclosure may, for example, be substantially free of other isomers, or may be admixed, for example, as racemates or with all other, more than one other or other selected stereoisomers.

To the extent that tricyclic sesquiterpene lactone derivatives of the formula I and salts thereof may exist in their tautomeric form, all such tautomeric forms are contemplated herein as part of the present disclosure.

Chemical abstract service registry numbers which are presented together with compound names do not reflect all possible stereochemical variations. They are thus to be understood as unambiguous identifier of the generalized stereochemical basic structures. All stereoisomers (that is, isomers other than constitution isomers) are encompassed as mentioned above; thus these numbers are not limiting the scope of the disclosure, but may represent preferred variants.

Where salt-forming groups (e.g. acidic groups, such as phenolic OH groups, or basic groups, such as amino or imino groups) are present within them, the tricyclic sesquiterpene lactone derivatives of the formula I may be in the free form or in the form of salts. The term "salt(s)", as employed herein, denotes acidic and/or basic salts formed with inorganic and/or organic acids and bases. In addition, when a tricyclic sesquiterpene lactone derivative of the formula I contains both a basic moiety and an acidic moiety, "inner salts" may be formed and are included within the term "salt(s)" as used herein. Pharmaceutically (or nutraceutically) acceptable (i.e., non-toxic, physiologically acceptable) salts are preferred, although other salts are also useful, e.g., in isolation or purification steps which may be employed during preparation. Salts of the tricyclic sesquiterpene lactone derivatives of the formula I may be formed, for example, by reacting a tricyclic sesquiterpene lactone derivative of the formula I with an amount of acid or base, such as an equivalent amount, in a medium such as one in which the salt precipitates or in an aqueous medium followed by lyophilization. Salts of the tricyclic sesquiterpene lactone derivatives of the formula I may also be formed by reacting a tricyclic sesquiterpene lactone derivative of the formula I with an alkylating agent, for example, by quarternization of an amine, where natural compounds are preferred. Also ion exchangers can be used to form salts from free forms or free forms from salts of a tricyclic sesquiterpene lactone derivative of the formula I.

Tricyclic sesquiterpene lactone derivatives of the formula I which contain a basic moiety, may form salts with a variety of organic and inorganic acids. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethanesulfonates, fumarates, glucoheptanoates, glycerolphos-phates, hemisulfates, heptanoates, hexanoates, hydrochlorides, hydrobromides, hydro-iodides, 2 hydroxyethanesul-fonates, lactates, maleates, methanesulfonates, 2-naphtalene-sulfonates, nicotinates, nitrates, oxalates, pectinates, per-sulfates, 3- phenylpropionates, phosphates, picrates, pivalates, propionates, salicylates, succinates, sulfates, sulfonates, tartrates, thiocyanates, toluenesulfonates, such as tosylates, undecanoates, and the like.

The tricyclic sesquiterpene lactone derivatives of the formula I which contain an acidic moiety may form salts with a variety of organic and inorganic bases. Exemplary basic salts include ammonium salts, alkali metal salts such as sodium, lithium, and potassium salts, alkaline earth metal salts such as calcium and magnesium salts, salts with organic bases (for example, organic amines) such as benzathines, dicyclohexylamines, N-methyl-D-glucamines, N-methyl-D-glucamides, t-butyl amines, and salts with amino acids such as arginine, lysine and the like. Also salts with salt-forming pharmaceutical and/or nutraceutical carrier materials are possible and encompassed by the disclosure.

Further, the tricyclic sesquiterpene lactone derivatives of the formula I may be in the form of their solvates, such as hydrates, of these derivatives.

Esters obtained according to methods known to the person skilled in the art and obtainable by trivial methods, e.g. reaction with organic carboxylic or sulfonic (e.g. C₁-C₂₀-)acids or activated derivatives thereof (e.g. in the form of halogenides, symmetric or asymmetric anhydrides or nitrophenol esters or by activation *in situ* using customary agents, e.g. triazole derivatives such as HATU or the like).

Within the present disclosure, the term "compound(s) of the formula I" is often used instead of "tricyclic sesquiterpene lactone derivative(s) of the formula I".

LDL refers to low density lipoprotein, HDL to high density lipoprotein, respectively.

"Obtainable" means that a product (e.g. extract or compound) may be obtained, preferably it is obtained by the specified method.

As "alkyl", methyl, ethyl, n-propyl, iso-propyl, n-butyl, iso-butyl, sec-butyl, tert.-butyl, n-pentyl or iso-pentyl can be especially preferred.

"Substituents" (also in the case of grammatically derived forms of this word (e.g. "substituted", e.g. in substituted aryl or the like) can especially be selected from the group consisting of C₁-C₇-alkyl, hydroxy, C₁-C₇-alkoxy, C₁-C₇-alkanoyloxy, C₁-C₇-alkoxycarbooxy, C₁-C₇-alkanesulfonyloxy, phenyl-C₁-C₇-alkoxy, amino, N-mono- or N,N-di-(C₁-C₇-alkyl, C₁-C₇-alkanoyl, C₁-C₇-alkoxycarbonyl, C₁-C₇-alkanesulfonyl and/or phenyl-C₁-C₇-alkyl)-amino, carboxy, C₁-C₇-alkoxycarbonyl, carbamoyl, N-mono- or N,N-di-(C₁-C₇-alkyl)-carbamoyl, sulfamoyl, N-mono- or N,N-di-(C₁-C₇-alkyl)-sulfamoyl and cyano. Preferably, in the case where one or more substituents are present, one or more, more preferably up to three, e.g. one or two such substituents, independently selected from the mentioned group, are present on the substituted moiety.

"Aryl" is preferably C₆-C₁₄-aryl, especially phenyl or naphthyl.

Where one or more, preferably 1 or 2, "heteroatoms" are present, they either are present instead of a ring carbon atom in the case of cyclic moieties (aryl, cycloalkyl) or instead of a chain carbon atom (in the case of acyclic moieties).

For the purpose of the specification, "carbohydrate" or "C₅₋₁₂-carbohydrate" refer to a mono or disaccharide consisting of one or two pentoses (C₅-carbohydrate or C₁₀-carbohydrate) and/or one or two hexoses (C₆-carbohydrate or C₁₂-carbohydrate), each optionally in their desoxy forms, the disaccharides in each form connected to each other via a glycosidic bond, unsubstituted or substituted with one, two, three, four or five substituents independently selected from the group consisting of methyl, ethyl, acetyl, benzoyl or 3,4,5-trihydroxybenzoyl. Examples of preferred pentoses are xylose, arabinose, each in the pyranosidic or furanosidic form. Examples of preferred hexoses are glucose, 6-deoxyglucose, rhamnose, each in the pyranosidic of furanosidic form. Examples of preferred glycosidic connections are 1→4 and 1→6. "Carbohydrate" or "C₅₋₁₂-carbohydrate" residues are bound to the higher general formula via one of its oxygen atoms.

Wherever in the present disclosure a compound of the formula I, a mixture of compounds of the formula I or one or more compounds of the formula I are mentioned, this intends to include the free (enriched or (at least substantially) pure) form (one preferred embodiment) and/or one or more (especially pharmaceutically or nutraceutically salts (alone or together referred to as pharmaceutically acceptable salts hereinafter) (another preferred embodiment)) where salt-forming groups (e.g. phenolic OH-groups or amino or basic imino groups) are present, (a) solvate(s) (yet another preferred embodiment), (an) ester(s) (meaning e.g. C₁-C₆alkanoyl, C₃-C₆alkenoyl, hydroxy-C₁-C₆alkanoyl or C₃-C₆alkenoyl esters, where one or more of the hydroxy groups in the corresponding sesquiterpenoid can be esterified) (another preferred embodiment) and/or (a) tautomer(s) (where tautomerism, e.g. of the oxo/enol type, is possible), or mixtures of two or more of these specific forms. A mixture may be the result of an extraction and/or of admixing two or more compounds of the formula I.

Where ratios of components are given in %, this means weight %, if not indicated otherwise.

Preferably, the total weight share of the compound or compounds of the formula I in an extract or mixture of compounds of the formula I or a purified compound of the formula I that is of USE according to the disclosure in the final extract, mixture or compound (direct or further enriched) is in the range from 0,01 to 100 % by weight, more preferably from 0,02 to 95 %, most preferably 0,05 to 95 %, from 0.05 to 50 % or e.g. from 0.1 to 90 %.

"One or more compounds of the formula I" or "a mixture of compounds of the formula I" means that either only one compound (in substantially pure form or as a direct extract or a further enriched extract) or a mixture of two or more compounds of the formula I (which mixture is preferred) can be present in an extract or pharmaceutical/nutraceutical formulation according to the disclosure or be of USE according to the disclosure.

By the term "regulation of body weight and/or fat loss" and/or "management of obesity" (especially leading to weight loss, that is, preferably use for body weight/body fat reduction or for treating, including preventing, obesity or optimisation of body composition), there is especially meant that by administration of one or more compounds of the formula I or preferably an (especially further enriched) extract comprising one or preferably two or more compounds of the formula I either a lower weight gain can be observed with the same offered diet, e.g. when compared with a control without such administration, or preferably a weight loss can be observed. More preferably, the weight loss is due to a reduction of the body fat. Thus, most preferably, the treatment of obesity is meant, either prophylactically to avoid a weight gain or preferably to reduce the body weight, especially to reduce the body fat. The term "prophylactically", in addition to treatment if a risk is present, also implies a generally healthy nutrition with an effect also in the healthy organism, e.g. in relation to elder people, e.g. in the sense of a maintenance of an appropriate, especially low, Body Mass Index (BMI). The regulation of body weight and/or fat loss is typically achieved by mechanisms other than suppression of the amount of food intake when compared with controls not receiving a treatment according to the disclosure. Preferably it is caused by increase of the metabolic rate over controls without treatment.

In some embodiments, the methods are direct to a subject which suffers from obesity, an obesity-related disorder, an obesity related disease, an obesity-related condition, diabetes, insulin-resistance syndrome, lypodystrpohy, nonalcoholic steatohepatitis, a cardiovascular disease, polycystic ovary syndrome, metabolic syndrome or a desire to lose body weight.

The present disclosure, in addition or as alternative to the regulation of body weight and/or fat loss and/or for the management of obesity, also comprises the USE for improving the total cholesterol HDULDL ratio (especially meaning raising the proportion of "good cholesterol" (HDL) to "bad cholesterol" (LDL)) in comparison (increasing the HDULDL ratio) to that without treatment, e.g. determined as described in the Examples or by standard blood parameter determination methods.

Thus also treatment of the symptoms of syndrome X corresponding to hyperlipidemia, obesity and inappropriate HDL/LDL ratio is possible:
Syndrome X, also named as Metabolic Syndrome, was first described by Reavan and often called Reavan-Syndrom (Reavan GM, Diabetes (1998) 37: 1595), and is defined by various organizations: International Diabetes Federation (IDF) (IDF Communications, Belgium, "The IDF consensus worldwide definition of the METABOLIC SYNDROME"; see http://www.idf.org/webdata/docs/IDF Meta def final.pdf), World Health Organization (WHO) (Khalib OMN, EMRO Technical Publications Series (2006) ISBN 978-92-9021-404-5, p 22), European Group for the Study of Insulin Restistance (EGIR) (Bar; Diabetes, Stoffwechsel und Herz (2007) 5: 329-334), US National Cholesterol Education Program (NCEP) (Circulation (2002) 106: 3143-3421) and by the American Heart Association (Grundy et al., Circulation (2004) 109: 433-438; (2005) 112: e285-e290) which are referring to risk parameters which are defined by different critical values.

Within the scope of the present disclosure , one, two or three of the defined risk parameters (also called "symptoms") as mentioned above can be reduced.

Although the patient may not notice any symptoms from metabolic syndrome, the attending physician could identify the following as signs of the condition: (1) elevated insulin levels, due to insulin resistance; (2) type II diabetes; (3) *central obesity* (a disproportionate amount of body fat in the abdominal region); (4) *hyperlipidemia* (high levels of fats (lipids) in the blood, *which include LDL ("bad") cholesterol* and triglycerides. In addition, the size of the LDLs may be smaller than usual, which is more likely to promote atherosclerosis); (5) *low level of HDL ("good") cholesterol*; (6) hypertension (high blood pressure); (7) elevated levels of blood factors that promote blood clotting, such as plasminogen activator inhibitor - 1 {PAI-I) and fibrinogen; (8) hyperuricemia (high levels of uric acid in the blood); and (9) microalbuminuria (small amounts of the protein albumin, found on urine tests) (Grundy S.M., Am. J Cardiol. 83: 25F29F, 1999).

The compounds of the formula I, or their mixtures (including extracts comprising them), may be used in non-therapeutic (e.g. cosmetic) treatments ("non-therapeutic use"), e.g. to enhance the fat distribution for aesthetic effects, e.g. the fat distribution at limbs, the abdomen and/or the buttocks, or for therapeutic and/or prophylactic purposes, e.g. to amend the health or cure certain disease symptoms or a whole disease including a syndrome, or to prevent certain dangers (such as diabetes development or cardiac or other infarction) to health. The disclosure thus in one embodiment also comprises the use of compounds of the formula I, mixtures thereof and/or extracts comprising them, especially the preferred compounds or compound, in the cosmetic treatment of the body of a warm-blooded animal, which (also if used elsewhere in this disclosure) especially means a human.

As used herein, the term "therapeutically effective amount" means the amount of the active compounds in the composition that will elicit the biological or medical response in a tissue, system, subject, or human that is being sought by the researcher, veterinarian, medical doctor or other clinician, which includes alleviation of the symptoms of the disorder being treated up to and including complete cure, e.g. from obesity. The novel methods of treatment of this disclosure are for disorders known to those skilled in the art.

As used herein, the term "prophylactically effective amount" means the amount of the active compounds in the composition that will elicit the biological or medical response in a tissue, system, subject, or human that is being sought by the researcher, veterinarian, medical doctor or other clinician, to prevent the onset of obesity or an obesity-related disorder, condition or disease in subjects as risk for obesity or the obesity-related disorder, condition or disease.

For testing, it is possible to conduct clinical trials (or animal assays as described in the Examples). e.g. clinical trials with humans (or other animals) analogous to those described in WO 2004/096252 or WO 2004/082700 , but only using one or more compounds of the formula I as described for the present disclosure.

The extracts or compounds according to the disclosure may be used as such, in the form or pharmaceutical or nutraceutical formulations (the latter term including food additives = supplements) or in the form of functional food.

Where the compounds or mixture of compounds of the formula I are used as supplement, this means that the compound(s) or a pharmaceutical or nutraceutical formulation comprising them can be added to any other nutrient or pharmaceutical or nutraceutical, preferably other than. Thus they can especially serve as food supplement. However, the compound(s) or formulations may also be administered as such.

The activity against obesity can, for example, be tested as described in the Examples, especially in the experiments with mice or rats described there.

"Nutraceuticals", "Functional Food", or "Functional Food products" (sometimes also called "Foodsceuticals", "Medicinal Food" or "Designer Food") for USE according to the present disclosure are defined as food products (including beverages) suitable for human consumption - the expression comprises any fresh or processed food having a health-promoting and/or disease-preventing property beyond the basic nutritional function of supplying nutrients, including food made from functional food ingredients or fortified with health-promoting additives, especially with effects in the prophylaxis or treatment of obesity, especially allowing for body weight reduction and/or body weight maintenance, appetite suppression, the provision of satiety or other changes in metabolism, and in which a compound or compound mixture of compounds of formula I, respectively, according to the disclosure is used as an ingredient (especially additive) as health benefit agent, especially in an effective amount.

"Comprising" or "including" or "having" wherever used herein is meant not to be limiting to any elements stated subsequently to such term but rather to encompass one or more further elements not specifically mentioned with or without functional importance, that is, the listed steps, elements or options need not be exhaustive. In contrast, "containing" would be used where the elements are limited to those specifically after "containing".

Where "about" is used or a specific numerical value is given without explicitly mentioning "about", this preferably means that a given value may deviate to a certain extent from the value given, e.g. in one of the embodiments by ± 20 % of the given numerical value, in another embodiment by ± 10 %.

While "obesity" is generally defined as a body mass index (BMI) over 30, for purposes of this disclosure, any subject, including those with a BMI of less than 30, who needs or wishes to reduce body weight or prevent body weight gain is included in the scope of "obese." Thus, subjects with a BMI of less than 30 and 25 and above (considered overweight) or below 25 are also included in the subjects of the disclosure. Morbid obesity refers to a BMI of 40 or greater.

By "metabolic rate" is meant the amount of energy liberated/expended per unit of time. Metabolism per unit time can be estimated by food consumption, energy released as heat, or oxygen used in metabolic processes. It is generally desirable to have a higher metabolic rate when one wants to loose weight. For example, a person with a high metabolic rate may be able to expend more energy (e.g., the body bums more calories) to perform an activity than a person with a low metabolic rate for that activity.

As used herein, "lean mass" or "lean body mass" refers to muscle and bone. Lean body mass does not necessarily indicate fat free mass. Lean body mass contains a small percentage of fat (roughly 3%) within the central nervous system (brain and spinal cord), marrow of bones, and internal organs. Lean body mass is measured in terms of density. Methods of measuring fat mass and lean mass include, but are not limited to, underwater weighing, air displacement plethysmograph, x-ray, DEXA scans, MRIs and CT scans. In certain embodiments, fat mass and lean mass is measured using underwater weighing as known in the art.

By "fat distribution" is meant the location of fat deposits in the body. Such locations of fat deposition include, for example, subcutaneous, visceral and ectopic fat depots.

By "subcutaneous fat" is meant the deposit of lipids just below the skin's surface. The amount of subcutaneous fat in a subject can be measured using any method available for the measurement of subcutaneous fat. Methods of measuring subcutaneous fat are known in the art, for example, those described in U.S. Patent No. 6,530,886.

By "visceral fat" is meant the deposit of fat as intra-abdominal adipose tissue. Visceral fat surrounds vital organs and can be metabolized by the liver to produce blood cholesterol. Visceral fat has been associated with increased risks of conditions such as polycystic ovary syndrome, metabolic syndrome and cardiovascular diseases.

By "ectopic fat storage" is meant lipid deposits within and around tissues and organs that constitute the lean body mass (e.g., skeletal muscle, heart, liver, pancreas, kidneys, blood vessels). Generally, ectopic fat storage is an accumulation of lipids outside classical adipose tissue depots in the body.

The functional food products or pharmaceutical products may be manufactured according to any suitable process, preferably comprising extraction of one or more compounds of the formula I and admixing to a functional food product or at least one nutraceutically or pharmaceutically acceptable carrier.

A functional food or a pharmaceutical or nutraceutical formulation comprising a compound, more preferably a compound mixture, for USE according to the present disclosure, can, for example, be obtained by
(a) extraction of one or more compounds and/or mixture of compounds of the formula I from one or more plants of the genera *mentioned above;,* and
(b) mixing the resulting one or more compounds and/or mixtures of compounds as active ingredient in the preparation of the functional food product with the other constituents thereof or in order to obtain a pharmaceutical or nutraceutical formulation with one or more carrier materials or with a solvent, e.g. water or an aqueous solvent (e.g. to give a juice or dispersion or solution).

Further processing steps may precede and/or follow, such as drying (e.g. freeze-drying, spray-drying and evaporation), granulation, agglomeration, concentrating (e.g. to syrups, formed via concentration and/or with the aid of thickeners), pasteurizing, sterilizing, freezing, dissolving, dispersing, filtering, centrifuging, confectioning, and the like.

When one or more compounds and/or a compound mixture according to the disclosure are added to a food product or pharmaceutical or nutraceutical, this also results in a functional food product or pharmaceutical or nutraceutical formulation according to the disclosure.

Preferably, a functional food product according to the disclosure comprises 0,01 to 30, e.g. 0,02 to 20, such as preferably 0.05 to 5, weight-% of a compound or mixture of compounds of the formula I according to the disclosure, the rest being food and/or nutraceutically acceptable carriers and/or customary additives.

Further additives may be included, such as vitamins, minerals, e.g. in the form of mineral salts, unsaturated fatty acids or oils or fats comprising them, other extracts, or the like.

The functional food products according to the disclosure may be of any food type. They may comprise one or more common food ingredients in addition to the food product, such as flavours, fragrances, sugars, fruit, minerals, vitamins, stabilisers, thickeners, dietary fibers, protein, amino acids or the like in appropriate amounts, or mixtures of two or more thereof, in accordance with the desired type of food product.

Examples of basic food products and thus of functional food products according to the disclosure are fruit or juice products, such as orange and grapefruit, tropical fruits, banana, apple, peach, blackberry, cranberry, plum, prune, apricot, cherry, peer, strawberry, marionberry, black currant, red currant, tomato, vegetable, e.g. carrot, or blueberry juice, soy-based beverages, or concentrates thereof, respectively; lemonades; extracts, e.g. coffee, tea, green tea; dairy type products, such as milk, dairy spreads, quark, cheese, cream cheese, custards, puddings, mousses, milk type drinks and yoghurt; frozen confectionary products, such as ice-cream, frozen yoghurt, sorbet, ice milk, frozen custard, water-ices, granitas and frozen fruit purees; baked goods, such as bread, cakes, biscuits, cookies or crackers; spreads, e.g. margarine, butter, peanut butter honey; snacks, e.g. chocolate bars, muesli bars; pasta products or other cereal products, such as muesli; ready-to-serve-dishes; frozen food; tinned food; syrups; oils, such as salad oil; sauces, such as salad dressings, mayonnaise; fillings; dips; chewing gums; sherbet; spices; cooking salt; instant drink powders, such as instant coffee, instant tee or instant cocoa powder; instant powders e.g. for pudding or other desserts; meat fish or fish or meat products, such as sausages, burgers, meat loafs, meatballs, meat extracts, canned or tinned fish or meat, meat vol-au-vent, meat or fish soup, meat or fish skewers, fish fingers; or the like.

One or more other customary additives may be present, such as flavour, fragrances or other additives, such as one or more selected from stabilizers, e.g. thickeners; colouring agents, such as edible pigments or food dyes; bulking agents, such as fruit pulp, e.g. in dried form; polyols, such as xylitol, mannitol, maltitol or the like; preservatives, such as sodium or potassium benzoate, sodium or calcium carbonate or other food grade preservatives; antioxidants, such as ascorbic acid, carotionoids, tocopherols or polyphenols; mono-, oligo-or polysaccharides, such as glucose, fructose, sucrose, soy-oligosaccharides, xylo-oligosaccharides, galacto-oligosacharides; other artificial or natural non- or low-caloric sweeteners, such as aspartame or acesulfame; bitterness blockers; acidifiers in the form of edible acids, such as citric acids, acetic acid, lactic acid, adipic acid; flavours, e.g. artificial or natural (e.g. botanical flavours); emulsifiers; thiols, e.g. allylic thiols; diluents, e.g. maltodextrose; wetting agents, e.g. glycerol; stabilizers; coatings; isotonic agents; absorption promoting or delaying agents; and/or the like.

The one or more compounds of the formula I or compound mixtures thereof according to the disclosure can also be comprised in confectioned formulations to be added to foods including beverages, e.g. in the form of powders or granules, e.g. freeze-dried or spray-dried, concentrates, solutions, dispersions or other instant form, or the like.

The pharmaceutical or nutraceutical formulation(s) (= composition(s), also for non-therapeutic use) according to the present disclosure can be prepared in various forms, such as granules, tablets, pills, syrups, solutions, dispersions, suppositories, capsules, suspensions, salves, lotions and the like. Pharmaceutical grade or food grade organic or inorganic carriers and/or diluents suitable for oral and topical use can be used to formulate compositions containing the therapeutically-active compounds. Diluents known in the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The compositions may also include one or more of the following: carrier proteins such as serum albumin; buffers; fillers such as microcrystalline cellulose, lactose, corn and other starches; binding agents; sweeteners and other flavouring or fragrancing agents; colouring agents; and polyethylene glycol. Those additives are well known in the art, and are used in a variety of formulations.

By "administered" herein is meant administration of a prophylactically and/or therapeutically effective dose of a tricyclic sesquiterpene lactone derivative of the formula I or a mixture of compounds of the formula I to an animal, especially a patient. By "therapeutically effective dose" herein is meant a dose that produces the effects, for which it is administered, especially a reduction of weight, more especially due to body fat reduction.

Preferably, the dosage of the compound or compounds of the formula I, based on the total weight of the compound(s) of the formula I, in both nutraceutical (including use as supplement) or pharmaceutical use typically is such that the amount of the compound(s) of the formula I administered to a patient is such that it is effective to achive the effects for which it is administered, or preferably a daily dose of about 0.2 to 200 g, e.g. in one embodiment of 0.5 to 7 g, or in another embodiment of 0,1 to 10 g, is administered to a person with a weight of 70 kg per day in one or more, e.g. 1 to 3, dosages (children/persons with differing weights receive a correspondingly (e.g. proportionally to the weight) modified dosage).

A warm-blooded animal or human, especially being a "patient" or "subject" for the purposes of the present disclosure, includes especially humans and further other (especially warm-blooded) animals. Thus, the tricyclic sesquiterpene lactone derivative(s) of the formula I or a mixture of compounds of the formula I, are applicable to both humans and animals. In the preferred embodiment the patient is a human. The patients will be treated either in prophylactic or therapeutic intention, the latter e.g. to avoid regain in weight after a weight (especially body fat) reduction (e.g. to avoid the yo-yo effect), or to avoid weight gain (especially due to an increase in body fat) *ab initio.*

Typically, the tricyclic sesquiterpene lactone derivative(s) of the formula I or a mixture of compounds of the formula I, having **THERAPEUTICAL** activity mentioned hereinbefore (e.g. weight control, weight loss, body fat reduction, and/or agonistic activity on the liver X receptor) may be administered with at least one physiologically (= pharmaceutically or nutraceutically) acceptable carrier to a patient, as described herein. The total concentration of therapeutically active tricyclic sesquiterpene lactone derivative(s) of the formula I or a mixture of compounds of the formula I in the formulation may vary from about 0.001-100 wt %, e.g. from 0,1 to 50 % by weight, the rest being the carrier material(s) and/or customary additives.

The tricyclic sesquiterpene lactone derivatives of the formula I or a mixture of compounds of the formula I may be administered alone or in combination with other treatments, i.e., other anti-obesity agents, common diets or the like.

"Combination" does not necessarily mean a fixed combination but may also mean that the compound(s) of the formula I may be administered in a chronically staggered manner with the combination partner(s), e.g. a combination product in the form of a kit of parts (which also is an embodiment of the disclosure) with other combination partners, other than those excluded hereinbefore. Preferably, the chronically staggered administration takes place such that the combination partners mutually influence, especially intensify (e.g. by way of an additive or preferably synergistic effect) their therapeutic efficiency.

Among other anti-obesity agents that may be combined, antilipidemics, e.g. atorvastatin, cerivastatin, fluvastatin, lovastatin, pravastatin, rosuvastatin, simvastatin, anti-obesity drugs, such as suppressants of the appetite, stimulators of the body's metabolism, or drugs or compositions interfering with the body's ability to absorb specific nutrients, such as sibutramine, diethylpropion, phendimetrazine, phentermine, fenfluramine, sibutramine, lipase inhibitors, such as orlistat; anorectics, such as dexedrine; cannabinoid receptor antagonists, such as rimonabant; acarbose; or the like, can be mentioned. Other helpful drugs or active agents may be administered, e.g. psychoactive agents, agents that help in the treatment of addictive behaviour, e.g. nicotine addiction, or the like, especially in so far as they help to support the prophylaxis or treatment according to the disclosure intended.

Weight loss diets, such as food combining, Hay diet, Atkins diet (low-carbohydrate diet), cabbage soup diet, diabetic diet, fat resistance diet, slimming world diet, low-fat diet, Pritkin diet, low-carbohydrate diet, low protein diet, negative calorie diet, raw food diet, weight watchers diet are possible examples of appropriate diets.

The tricyclic sesquiterpene lactone derivatives of the formula I or a mixture of compounds of the formula I, itself or as mixtures of certain complexity, e.g. extracts or preparations, e.g. juices etc of the above mentioned plants, of this disclosure are particularly useful for controlling the body weight, preferably the treatment of obesity (adipositas).

Natural compounds of the formula I, or extracts comprising one or more thereof, for USE according to the present disclosure are isolated from one or more plants of the genera listed above.

Plant parts are, e.g., leaves, bark, flowers, buds, fruits, stems, shoots, roots, tubers or other parts of plants, and they or the plants can be complete, hackled, crushed, chopped up, broken up, homogenized, dried, fermented or treated otherwise.

By the term "extract", either a direct extract (in liquid or preferably dried form), e.g. obtained as described below, or preferably a further enriched extract (obtainable e.g. by one or more further purification steps after extraction, e.g. chromatography, for example as described below) containing one or more, preferably two or more compounds of the formula I is meant.

The compound(s) of the formula I in the form of an extract and extracts according to the disclosure can be obtained by extraction of plants or plant parts from a plant of the family of Asteracea, e.g. plants or plant parts of a plant of the species *Cynara,* more especially from *Cynara scolymus* or parts thereof.

The tricyclic sesquiterpene lactone derivatives of the formula I or a mixture of compounds of the formula I, or an extract comprising one or more of them, of the present disclosure can be prepared by extracting and preferably enriching up to isolating them from the plants or parts of plants. Auxiliary means such as (especially ultrasonic) sonication, heating (e.g. to temperatures from room temperature to 50 °C), stirring, re-extraction, evaporation or the like, may be used to allow for appropriate extraction.

Extraction preferably takes place with a non polar or more preferably a polar solvent or solvent mixture, e.g. water and/or an alcohol, such as methanol or ethanol, and/or with a liquid or superfluid gas, especially superfluid CO₂.

Optionally, the solvent may be removed after extraction.

Preferably, the extracts can subsequently be further enriched by one or more additional purification steps, such as distribution, e.g. between an aqueous and an ether (e.g. diethyl ether) phase for one or more times, precipitation (e.g. crystallisation) or especially chromatography, by which it is possible to obtain further enriched extracts or isolated compounds of the formula I.

The tricyclic sesquiterpene lactone derivatives of the formula I can e.g. be isolated or the extracts prepared as described in the appended examples. The method for detection can comprise high pressure liquid chromatography (HPLC) on reversed phase silica gel (C18) with water/ acetonitrile-gradient as an elution solvent with UV as well as MS detection which are used for the product analysis and production optimization. It will be clear to those having ordinary skill in this art that the dibenzo[a,c]cyclooctadiene derivatives of the formula I, though per se natural products, can alternatively be synthesized according to standard methods leading to compounds identical with the natural compounds, where appropriate methods, for example, can be deduced from the following publications: March's Advanced Organic Chemistry: Reaction, Mechanisms and Structure, 5th ed. by Michael B. Smith, Jerry March, Wiley-Interscience; 2001; Classics in Total Synthesis: Targets, Strategies, Methods by K. C. Nicolaou, E. J. Sorensen John Wiley & Son Ltd, 1996 and The Art and Science of Total Synthesis at the Dawn of the Twenty-First Century. Nicolaou KC et al., Angew Chem Int Ed Engl 2000, 39 (1): 44 -122.

Preferably, the compound or compounds, in the embodiments of the disclosure, are enriched in the mixtures or extract or purified extracts, or in another embodiment as single compound, to a percentage, in independent embodiments of the disclosure, of 10, 20, 30, 40, 50, 60, 70, 75, or (meaning in essentially pure form) 80, 85, 90, 92, 94, 95, 96, 97 or 98 % by weight of the complete extract or purified extract, respectively.

A process of preparing an extract containing a compound of the formula I, or a mixture of compounds of the formula I, at least in an amount of 10, 20, 30, 40, 50, 60, 70, 75, or (meaning in essentially pure form) 80, 85, 90, 92, 94, 95, 96, 97 or 98 % by weight of the complete extract or purified extract also is an embodiment of the present disclosure.

Where USE is mentioned, this especially refers to one or more of the following embodiments of the disclosure which can be inserted wherever USE is mentioned:
(1) A compound of the formula I, or a mixture of compounds of the formula I, for use in therapeutic (including prophylactic) treatment of an animal, preferably a mammal, especially a human, for the regulation of body weight and/or fat loss and/or for the management of obesity, especially for decreasing (= reducing herein) the body weight, more especially for decreasing the body fat and/or for improving the total cholesterol HDULDL ratio; or simply for maintenance of a healthy body, e.g. a low BMI.
(2) A pharmaceutical or nutraceutical composition comprising a compound of the formula I, or a mixture of compounds of the formula I, as active ingredient together with a pharmaceutically acceptable diluent or carrier, especially for use in the therapeutic and/or prophylactic treatment mentioned under (1).
(2') A pharmaceutical or nutraceutical composition for the treatment as mentioned under (1) comprising a compound of the formula I, or a mixture of compounds of the formula I, or especially a (preferably further enriched) extract comprising one or more compounds of the formula I, and a pharmaceutically acceptable diluent or carrier, as active ingredient supplement to a food.
(3) A functional food comprising a compound of the formula I, or a mixture of compounds of the formula I, or especially a (preferably further enriched) extract, as active ingredient for the treatment as mentioned under (1).
(4) A method for the treatment as mentioned under (1), especially any one or more of obesity, and/or excess body fat; and/or for improving the total cholesterol HDULDL ratio; in a subject in need of such treatment, respectively, comprising administering a pharmaceutically or nutraceutically effective amount of a compound of the formula I, a mixture of compounds of the formula I, as active ingredient, to an individual ("individual" meaning a warm-blooded animal, especially a human, wherever mentioned), especially to an individual in need thereof.
(5) The use of a compound of the formula I, or a mixture of compounds of the formula I, as active ingredient for the manufacture of a medicament or nutraceutical or food supplement for the treatment mentioned under (1).
(6) A method or use as defined under (4), comprising co-administration, e.g. concomitantly or in sequence, of a therapeutically effective amount of compound of the formula I, or a mixture of compounds of the formula I, as active ingredient and a different pharmaceutically active compound and/or a pharmaceutically acceptable salt thereof, said different pharmaceutically active compound and/or salt thereof being especially for use in the treatment as mentioned under (1).
(7) A combination product comprising a therapeutically effective amount of a compound of the formula I, or a mixture of compounds of the formula I, as active ingredient, and a different pharmaceutically active compound and/or a pharmaceutically acceptable salt thereof, said second pharmaceutically active compound being especially for use or of use in the treatment mentioned under (1).

The USE may also be for purely cosmetic purposes (or generally for non-therapeutic use as defined above), where in all embodiments of the disclosure, such as the above embodiments (1) to (7), "pharmacaeutical", "pharmaceutically", "nutraceutical" and "nutraceutically" are replaced with "cosmetic" or "cosmeticalls", respectively, thus provide in the corresponding embodiments for non-therapeutic use.

For any of the USEs, the USE is such that the compound(s) of formula I or mixtures thereof are the active ingredient, that is, they are already alone capable of achieving the intended effect (regulation of body weight and/or fat loss and/or management of obesity, especially decreasing of body weight, and/or more especially decreasing body fat) and are thus themselves the important active principle for the treatment(s) mentioned. Throughout the present specification, the prophylactic and/or therapeutic treatment or regulation of body weight and/or fat loss and/or management of obesity, especially decreasing of body weight, more especially decreasing body fat, are especially preferred embodiments according to the disclosure.

In any of the USEs mentioned, the compound(s) of the formula I may be present and/or administered in free form, in the form of a pharmaceutically and/or nutraceutically acceptable salt, in the form of tautomers, in the form of esters and/or in the form of solvates.

In addition or alternatively, the following combinations and their USE or USEs are preferably excluded from the present disclosure, though prior art does not explicitly mention that *Cynara scolymus* there is the principle active in the USEs according to the present disclosure:
Extracts prepared from *Cynara scolymus* with the addition of sulfated amino acids or suitable thio derivatives, especially cystein.

By "administering" herein is especially meant administration of a therapeutically or nutraceutically effective dose of a compound of the formula I, or a mixture of compounds of the formula I, to a cell either in cell culture or especially to an animal, especially a human patient. By "therapeutically or nutraceutically effective dose" herein is preferably meant a dose that produces the effects for which it is administered.

The pharmaceutical or nutraceutical preparations may be sterilized and/or may contain carrier materials or adjuvants such as preservatives, stabilizers, binders, disintegrants, wetting agents, skin or mucuous membrane penetration enhancers, emulsifiers, salts for varying the osmotic pressure and/or buffers, or other ingredients, excipients or carrier materials known in the art.

### Description of the figures

Fig 1: Graphic representation of the body weight gain in correlation to the control group, body weights of the control groups were normalized to 1.00 (weight loss in rats by administration of cynaropicrin).
Fig 2: Graphic Representation of the body fat gain (determined by DEXA) in correlation to the control group, body fat of the control groups were normalized to 1.00 (reduction of body fat in rats by cynaropicrin)
Fig 3: HPLC analysis of the enriched extract according example 4 A; ELSD upper line, UV (210 nm) lower line.
Fig 4: HPLC analysis of the final product according example 5 identified as Cynaropicrin (IMD-009047); ELSD upper line, ret time 9.372 min, UV (210 nm) lower line, ret time 9.235 min.
Fig. 5: Fig 3: HPLC analysis of an extract according example 4 B; UV (210 nm).

### Preferred embodiments of the disclosure

In one preferred embodiment, the disclosure relates to a USE of a compound of the formula I or a mixture of two or more such compounds, wherein
A1, A2 and A3 independently from each other represent the diradicals **CH₂, **CH-O-R1, or **C=O,
   with R1 denoting hydrogen, a straight-chain or branched-chain alkyl group having 1 to 12 carbon atoms, or a (-C(O)-(CH₂)ₙ-CRₐ=CRₐR_{b}) group wherein n is zero or an integer from 1 to 12 and
   Rₐ and R_{b} independently from each other are selected from a group Z, consisting of hydrogen and a straight-chain or branched-chain alkyl group having 1 to 6 carbon atoms that is unsubstituted or substituted by hydroxy, and
B1, B2 and B3 independently from each other represent the diradicals **C=CH₂, **CH-CH₂-R1*, and **COH-CH₂-O-R1 with R1 as defined above and R1* being R1 as defined above or a straight-chain or branched-chain alkoxy group having 1 to 12 carbon atoms,
where if one or more heteroatoms mentioned above are present they are present instead of one or more carbon atoms and are selected from the group consisting of S, N, NH, O, P and Se, preferably S, N, NH and O,
where the compound(s) of the formula I may be present in free form, in the form of a pharmaceutically and/or nutraceutically acceptable salt, in the form of tautomers, in the form of esters and/or in the form of solvates.

A preferred embodiment relates to a USE of one or more compounds selected from the group consisting of 3-Epi-11,13-dihydrodeacylcynaropicrin (99305-01-8), Subexpinnatin (81421-79-6), 11,13-Dihydrodeacylcynaropicrin (66761-12-4), 11beta,13-Dihydrocynaropicrin (160661-30-3), Isoamberboin (30825-69-5), 3,11,13-Trihydroxy-10(14)-guaien-12,6-olide (70894-20-1), Dehydrocynaropicrin (35821-02-4), Sibthorpin (94410-23-8), 8-Deoxy-11,13-dihydroxygrosheimin (83551-03-5), Isolipidiol (30825-68-4), 8-Hydroxy-3-oxo-4(15),10(14)-guaiadien-12,6-olide (38142-62-0), 3,8-Dihydroxy-10(14),11(13)-guaiadien-12,6-olide (84305-03-3), Grossheimin (22489-66-3), Integrifolin (89647-87-0), 8beta-Hydroxydehydrozaluzanin C (83991-71-3), Muricatin (52597-25-8), Cynaropicrin (35730-78-0), 13-Chloro-3,11-dihydroxy-4(15),10(14)-guaiadien-12,6-olide (142563-68-6), 3-Acetyl-13-chloro-13-deoxysolstitialin (142546-23-3), Cynaroside A (117804-06-5), 8-Deoxy-11-hydroxy-13-chlorogrosheimin (83551-02-4), Cynarascoloside A (518034-52-1), Cynarascoloside B (518034-53-2), Cynarascoloside C (518034-54-3), Cynarinin A (849146-43-6), Cynarinin B (849146-44-7), where the compound(s) of the formula I may be present in free form, in the form of a pharmaceutically and/or nutraceutically acceptable salt, in the form of tautomers, in the form of esters and/or in the form of solvates, where the given CAS numbers represent only preferred stereoisomeric forms, so that the given names can also represent other stereoisomeric forms, or mixtures of two or more such forms.

One or more of the structures of the disclosure presented in the following list are especially preferred for USE according to the disclosure:

| Names | | CAS-RN |
|---|---|---|
| Isolipidiol | 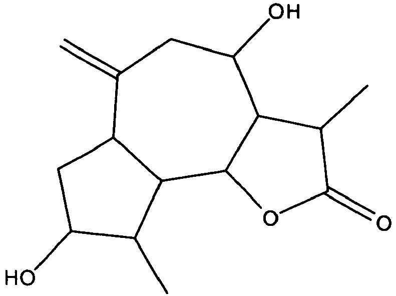 | 30825-68-4 |
| 3,8-Dihydroxy-10(14)-guaien-12,6-olide; (1alpha,3beta,4alpha,5alpha,6alpha ,8alpha,11alpha) | | |
| Dentalactone | | 155662-30-9 |
| 3,8-Dihydroxy-10(14)-guaien-12,6-olide; (1alpha,3beta,4beta,5alpha,6alpha, 8beta,11beta) | | |
| Isoamberboin | 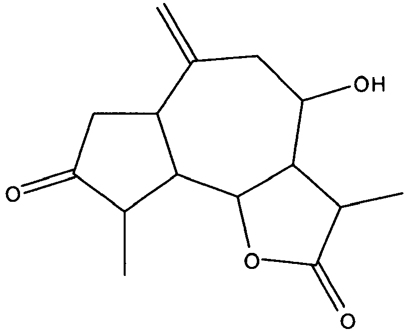 | 30825-69-5 |
| 3,8-Dihydroxy-10(14)-guaien-12,6-olide; (1 alpha,3beta,4alpha,5alpha,6alpha ,8alpha,11alpha); 3-Ketone | | |
| 8-Hydroxy-3-oxo-10(14)-guaien-12,6-olide | | 22339-28-2 |
| 3-Epi-11,13-dihydrodeacylcynaropicrin | 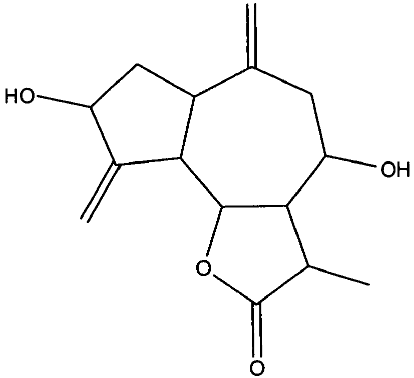 | 99305-01-8 |
| 3,8-Dihydroxy-4(15),10(14)-guaiadien-12,6-olide; (1alpha,3alpha,5alpha,6alpha,8alph a,11betaH) | | |
| 11,13-Dihydrodeacylcynaropicrin | | 66761-12-4 |
| 3,8-Dihydroxy-4(15),10(14)-guaiadien-12,6-olide; (1alpha,3beta,5alpha,6alpha,8alpha ,11betaH) | | |
| 3,8-Dihydroxy-4(15),10(14)-guaiadien-12,6-olide | | 35932-54-8 |
| 8alpha-Hydroxy-11alpha,13-dihydrozaluzanin C | | 84813-81-0 |
| 3,8-Dihydroxy-4(15),10(14)-guaiadien-12,6-olide; (1alpha,3beta,5alpha,6alpha,8alpha ,11alphaH) | | |
| 3,8-Dihydroxy-4(15),10(14)-guaiadien-12,6-olide; (1alpha,3beta,5alpha,6alpha,8beta,11a IphaH)-form | | |
| 8beta-Hydroxy-11beta,13-dihydrozaluzanin C | | 153753-47-0 |
| 3,8-Dihydroxy-4(15),10(14)-guaiadien-12,6-olide; (1alpha,3beta,5alpha,6alpha,8beta, 11betaH) | | |

| | | |
|---|---|---|
| 8-Hydroxy-3-oxo-4(15),10(14)-guaiadien-12,6-olide | 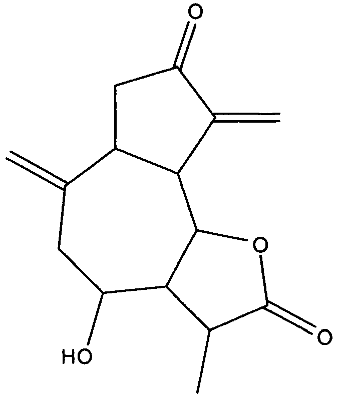 | 38142-62-0 |
| 3,8-Dihydroxy-4(15),10(14)-guaiadien-12,6-olide; (1 alpha,3beta,5alpha,6alpha,8alpha ,11betaH); 3-Ketone; 8-Hydroxy-3-oxo-4(15),10(14)-guaiadien-12,6-olide | | |
| 3,8-Dihydroxy-10(14),11(13)-guaiadien-12,6-olide | 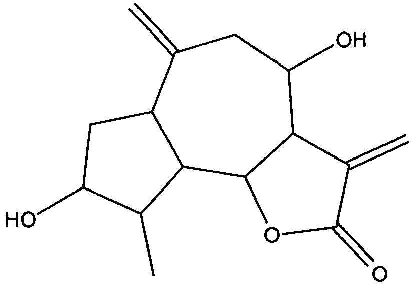 | 84305-03-3 |
| 3,8-Dihydroxy-10(14)-guaien-12,6-olide; (1alpha,3beta,4alpha,5alpha,6alpha,8b eta,11alpha)-form; 11,13-Didehydro | | 405283-65-0 |
| 8-Epigrosheimin | 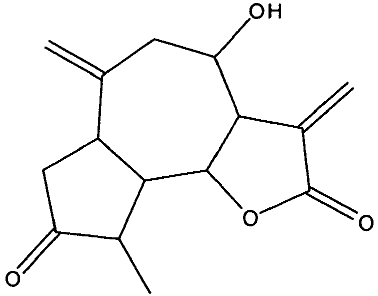 | - |
| 3,8-Dihydroxy-10(14)-guaien-12,6-olide; (1alpha,4alpha,5alpha,6alpha,8beta );11,13-Didehydro, 3-ketone | | |
| Grossheimin | | 22489-66-3 |
| 8-Hydroxy-3-oxo-10(14),11(13)-guaiadien-12,6-olide | | |

| | | |
|---|---|---|
| 3,8-Dihydroxy-4(15),10(14),11(13)-guaiatrien-12,6-olide; (1xi,3xi,5xi,6xi,8xi)-form | 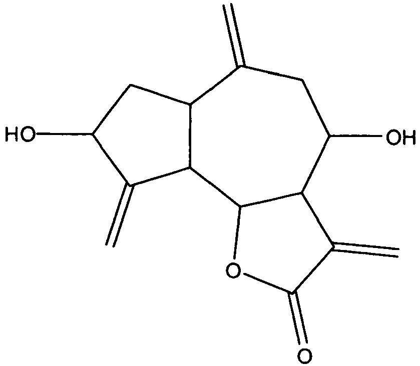 | - |
| Integrifolin | | 89647-87-0 |
| 3,8-Dihydroxy-4(15),10(14),11(13)-guaiatrien-12,6-olide; (1alpha,3beta,5alpha,6alpha,8beta) | | |
| 8-Hydroxyzaluzanin C | | 31565-50-1 |
| 3,8-Dihydroxy-4(15),10(14),11(13)-guaiatrien-12,6-olide; (1alpha,3beta,5alpha,6alpha,8alpha ) | | |
| 8beta-Hydroxydehydrozaluzanin C | 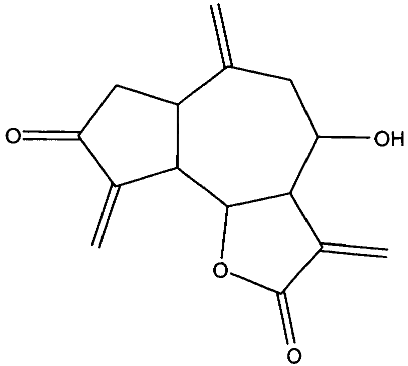 | 83991-71-3 |
| 3,8-Dihydroxy-4(15),10(14),11(13)-guaiatrien-12,6-olide; (1alpha,3beta,5alpha,6alpha,8beta); 3-Ketone | | |
| 8-Hydroxy-3-oxo-4(15),10(14),11(13)-guaiatrien-12,6-olide | | 67667-77-0 |
| Cynaratriol | 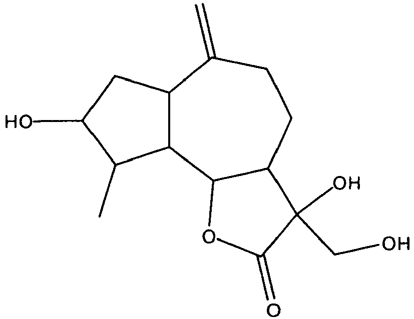 | 70894-20-1 |
| 3,11,13-Trihydroxy-4(15),10(14)-guaiadien-12,6-olide; (1alpha,3beta,5alpha,6alpha,11alph a); 4beta,15-Dihydro | | |

| | | |
|---|---|---|
| Sibthorpin | 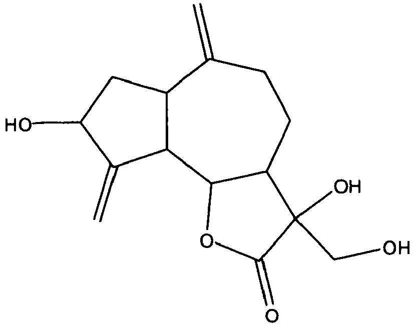 | 94410-23-8 |
| 3,11,13-Trihydroxy-4(15),10(14)-guaiadien-12,6-olide; (1alpha,3alpha,5alpha,6alpha,11alp ha) | | |
| Solstitialin | | 22738-70-1 |
| 3,11,13-Trihydroxy-4(15),10(14)-guaiadien-12,6-olide; (1alpha,3beta,5alpha,6alpha,11alph a) | | |
| 8-Deoxy-11,13-dihydroxygrosheimin | 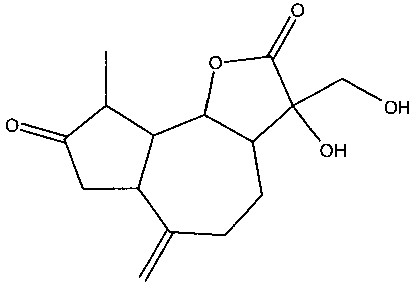 | 83551-03-5 |
| 11,13-Dihydroxy-3-oxo-10(14)-guaien-12,6-olide; (1alpha,4alpha,5alpha,6alpha,11xi) | | |
| 3,11,13-Trihydroxy-4(15),10(14)-guaiadien-12,6-olide; (1alpha,3beta,5alpha,6alpha,11alpha)-form; 4,15-Dihydro, 3-ketone | | |
| 11,13-Dihydroxy-3-oxo-10(14)-guaien-12,6-olide; (1alpha,4alpha,5alpha,6alpha,11alpha) -form | | 255722-99-7 |
| Muricatin | 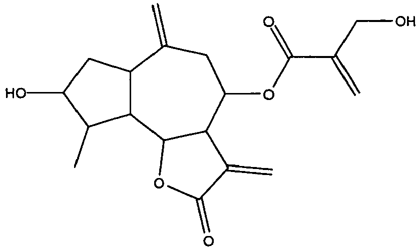 | 52597-25-8 |
| 3,8-Dihydroxy-10(14)-guaien-12,6-olide; (1alpha,3beta,4alpha,5alpha,6alpha ,8alpha, 11 alpha); 11,13-Didehydro, 8-O-(2-hydroxymethylpropenoyl) | | |
| Cynaropicrin | 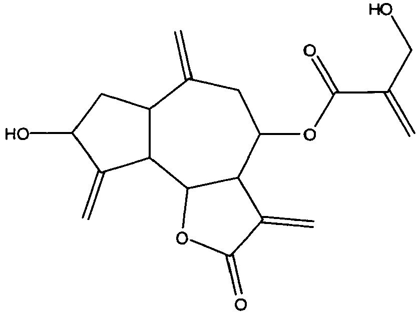 | 35730-78-0 |
| 3,8-Dihydroxy-4(15),10(14),11(13)-guaiatrien-12,6-olide; (1alpha,3beta,5alpha,6alpha,8alpha ); 8-O-(2-Hydroxymethyl-2-propenoyl) | | |
| Saupirin | | 35932-39-9 |
| 3,8-Dihydroxy-4(15),10(14),11(13)-guaiatrien-12,6-olide; (1xi,3xi,5xi,6xi,8xi); 8-O-(2-Hydroxymethylpropenoyl) | | |

Also preferred are the esters (meaning e.g. C₁-C₆alkanoyl, C₃-C₆alkenoyl, hydroxy-C₁-C₆alkanoyl or C₃-C₆alkenoyl esters) of the above shown non-acylated structures, especially but not limited to their ester presented as last compound in the above list.

The geometries of the double bonds within the structures shall not be assigned as been drawn. So they can be present either as E or Z configuration isomers, or as mixture of such isomers.

Especially preferred for USE according to the disclosure are Cynaropicrin (IUPAC name: (8-hydroxy-3,6,9-trimethylidene-2-oxo-3a,4,5,6a,7,8,9a,9b-octahydroazuleno[4,5-b]furan-4-yl) 2-(hydroxymethyl)prop-2-enoate), or Grossheimin IUPAC name: 4-hydroxy-9-methyl-3,6-dimethylene-3a,4,5,6a,7,9,9a,9b-octahydroazuleno[5,4-d]furan-2,8-dione), or a mixture of these two compounds.

The disclosure also relates to the USE according to the disclosure of any one of the compounds of the formula I mentioned in the examples, alone or in combination with one or more other compounds of the formula I mentioned therein.

### Examples

The present disclosure is further explained by the following examples. The specific examples which follow illustrate the methods in which the compositions of the present disclosure may be prepared, components therein and their use, as well as other embodiments of the disclosure, but are not to be construed as limiting the invention in scope.

### General Experimental Procedures:

If not mentioned otherwise, chemicals are obtained in analytical grade from Merck (Darmstadt, Germany) or Sigma-Aldrich (Deisenhofen, Germany). LC-MS analyses are performed using an Agilent HP1100 (Agilent, Waldbronn, Germany) liquid chromatograph coupled with a LCT mass spectrometer (Micromass, Manchester, UK) in the positive and negative electrospray ionisation (ESI) mode, based on slight modification of a previously described method [9]. A Waters symmetry column is used as stationary phase. Mobile phase A: 0.1 % Formic acid in water, mobile phase B: 0.1 % Formic acid in acetonitrile; gradient: 0-1 min. 100 % A, from 1-6 min. to 90 % B, from 6 to 8 min to 100 % B, from 8-10 min 100 % B. LC-MS spectra are recorded in the range of molecular weights between 150 and 1.600 U. HPLC-UV/Vis analyses are carried out on a HP 1100 Series analytical HPLC system (Agilent, Waldbronn, Germany) comprising a G 1312A binary pump system, a G 1315A diode array detector, a G 1316A column compartment, a G 1322A degasser and a G 1313A autoinjector. Mobile phase: A = 0.1 % Trifluoroacetic acid in water, B = 0.1 % Trifluoroacetic acid in acetonitrile. A Nucleodur® (Trademark by Macherey & Nagel) RP 18 column (125 x 4 mm, particle size 5 µm) serves as stationary phase. Aliquots of the samples (representing 2 - 10 µg of methanol-soluble materials, according to the concentrations of main metabolites) are analysed at 40°C with a flow of 1 ml/min in the following gradient: Linear from 0 % B to 100 % B in 20 min, thereafter isocratic conditions at 100% acetonitrile for 5 min; followed by regeneration of the column for 5 min. HPLC-UV chromatograms are recorded at 210 nm and 254 nm. Diode array detection (DAD) is employed to record HPLC-UV/Vis spectra in the range of 190 - 600 nm. The HP ChemStation® (Trademark by Agilent Technologies, Inc; see "Agilent" above) software allows for an automated search for calibrated standard compounds in crude extracts.

### Example 1: feeding experiments

Feeding experiments can be performed in any mammal and are done in different species.

For the present experiments, the compound to be tested is added to the feed of the individuals. This is beneficial against enforcement because it allows a validation of the acceptance of the test compounds. The experiments are performed with negative control and are correlated to a commercial slimming product.

Cynaropicrin is tested using six weeks old, none-adult, male Sprague-Dawley (SD) rats (Charles Rivers, Sulzfeld, Germany). Cynaropicrin is mixed with the chow (ssniff Spezialdiaten GmbH, Soest, Germany) to a final concentration of 0.1 % by weight and fed for 8 weeks in two different treatment regimes:
- Balanced diet (standardised to 4 % fat)
- High fat diet (standardised to 34 % fat)

Controls obtain the same diet without Cynaropicrin or other anti-obesity additives.

Body weight is studied and analysed every week, body fat and mineral bone density are analysed by DEXA (scanner, see Example 3) in week 0, in week 4 and at the last day of the study in week 8. Furthermore, the white body fat weight is determined after removal of the tissue at the end of the feeding study in week 8. For the DEXA measurements the same apparatuses and methodology are used as described in example 3.

The GE Lunar Piximus2 DEXA scanner (GE Healthcare, Munich, Germany) gives accurate information on differences in body composition in mice (see Nagy, TR and Clair, A-L. Precision and accuracy of dual-energy X-ray absorptiometry for determining in vivo body composition in mice. Obesity Res. 2000; 8:392-398; Brommage Am J Physiol Endocrinol Metab 285: E454-E459, 2003). Validation and calibration of DEXA body composition in mice; Sarah L. Johnston, Wendy L. Peacock, Lynn M. Bell, Michel Lonchampt, and John R. Speakman, Obesity Research 2005 13 (9), 1558). One scanning procedure takes up to five minutes per individual and provides data on fat mass, lean mass, bone mineral content and bone mineral density. The method is validated against Soxhlet (SOX) fat extraction in mice and a strong correlation (r 2 > 0.95) between fatDEXA and fatSOX is determined (published information).

As a control experiment within the high fat diet, epigallocatechin-3-gallate (EGCG, Chengdu Purification Technology Development Co. Ltd, Chengdu, China) in pure form is used as the standard for comparison of potency and differentiation as well as benchmark against competing compounds or formulations against obesity. Anti-obese and fat reducing effects are demonstrated for EGCG in animal studies and in human trials. EGCG is marketed as a functional food ingredient claiming an anti-obesity effect (Wolfram, S et al. Mol Nutr Food Res 2006; 50(2):176-87; Wolfram, S et al. Ann Nutr Metab 2005; 49(1):54-6, Klaus, S et al. Int J Obes 2005; 29(6):615-23; Hill, AM et al. J Am Coll Nutr. 2007; 26(4):396S-402S). The final concentration of EGCG used is 0.1 %, too.

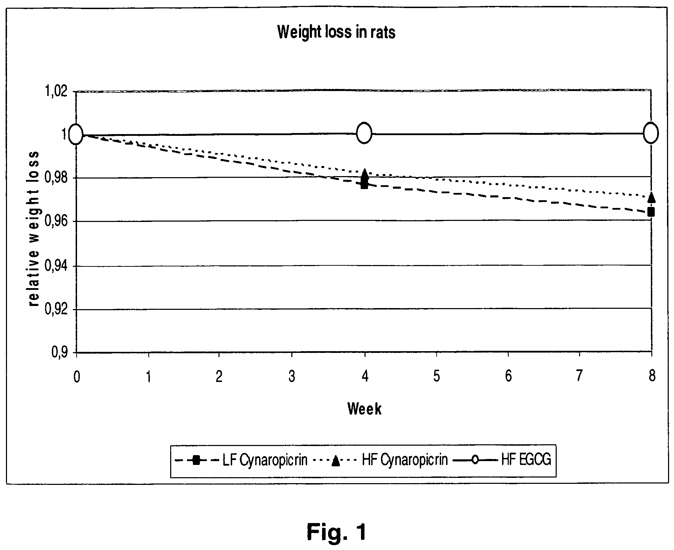

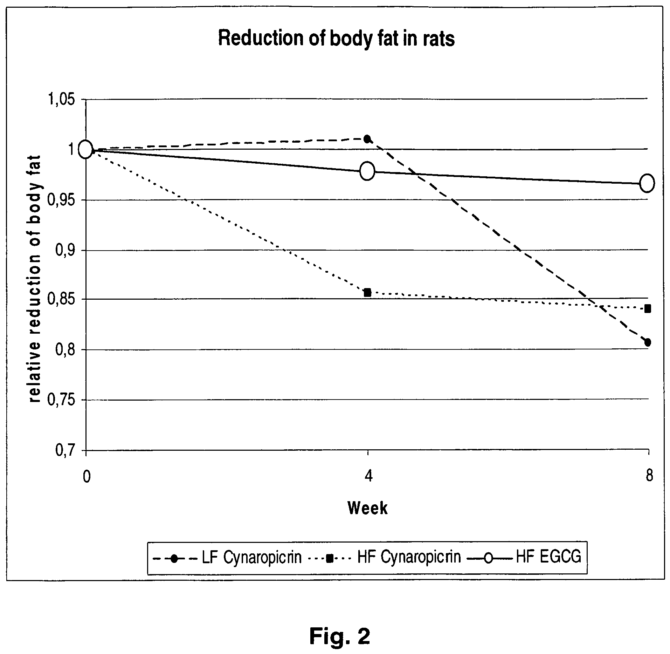

Result: Feeding of cynaropicrin results under high fat diet conditions in a reduced body weight accumulation and reduced body fat accumulation in comparison to the control groups. No effects are observed for EGCG in this study, as presented in figures 1 and 2.

Efficiency of assimilation is determined on the last 3 days and the first 3 days of begin to feed cynaropicrin as at the last 3 days of the feeding experiment in week 8.

Therefore the food intake and faeces are analysed and the energy content are determined by using a bomb calorimeter (IKA C7000). Efficiency of assimilation (in %) is calculated from the ratio between the energy consumption from food intake and the difference between energy intake and energy excretion.

Feeding of cynaropicrin has no influence on food intake in correlation to the control groups. Further the assimilation efficiency is the same for same types of diet:
LF control group: 87 %
LF cynaropicrin: 86 %
HF control group: 83 %
HF cynaropicrin: 83 %

Based on the same food intake and assimilation efficiencies reduction of body weight and body fat is most presumably caused by effects on energy metabolism. Application of Cynaropicrin results in a reduced body weight and body fat gain by identical energy intake.

### Example 2: clinical blood chemistry findings

The following parameters are involved in energy metabolism and thereby related to the metabolic syndrome. They are determined from blood samples taken in week 1; 4; and 8 of the rat study: Total cholesterol levels; HDL, LDL, TG (triglycerides), and glucose. Furthermore, the activity of liver enzymes such as ALT (alanin-aminotransferase), AST (aspartat-aminotransferase), and AP (alkaline phosphatase), as well as the concentration of Bilirubin, Albumin, Creatin Kinase, and electrolytes (Na+; K+; Ca++; Cl-) are determined as safety markers to exclude any toxic effects such as e.g. liver damage.

Blood samples are taken from the sublingual vein and collected in Li-Heparin coated vials. The blood samples are stored on ice for a short period of time before it is centrifuged for 15 minutes at 8°C and 3500 rpm. The supernatant plasma is transferred in a reaction tube and stored at -80°C until clinical chemistry measurements are performed. All measurements are conducted using an Olympus AU 400 at the gene centre of the LMU Munich, working group of Prof. Wolf. Statistical calculations are performed with Statistika (Statsoft (Europe) GmbH, Hamburg, Germany) und Sigma Stat (Jandel Scientific, San Raphael, CA, USA).

Electrolytes, bilirubin and liver enzymes such as e.g. AP are found to be statistically unchanged in all diet groups' indicating absence of any obvious toxic effects or liver damage.

Most importantly the data enables us to demonstrate an improved total cholesterol:HDL/LDL ratio for Cynaropicrin treated rats under high fat (HF) diet compared to the control group (HF only). Other than the atherogenic LDL, HDL is considered to act cardio-protective. Usually, a high HDULDL ratio ("More HDL than LDL") is considered as a beneficial blood parameter by physicians.

### Example 3: pathological parameters, clinical findings

Main organs are analysed macroscopically for safety reasons and to investigate the effects of the different diets on these organs (e.g. induction of cholestatic ("fatty") livers etc.). After organ removal, macroscopic inspections of e.g. heart, kidneys; GI tract, spleen, liver are performed.

Thereby, no significant alterations are found. Additionally, a histo-pathological analysis of the kidneys is done but does not result in any observation of an adverse effect on kidney morphology and function. Therefore it can be concluded that the observed biological activities of Cynaropicrin are most likely due to a real metabolic effect rather than being caused by simple toxic effect such as e.g. a test item related organ damage.

### Example 4: preparation of an enriched extract

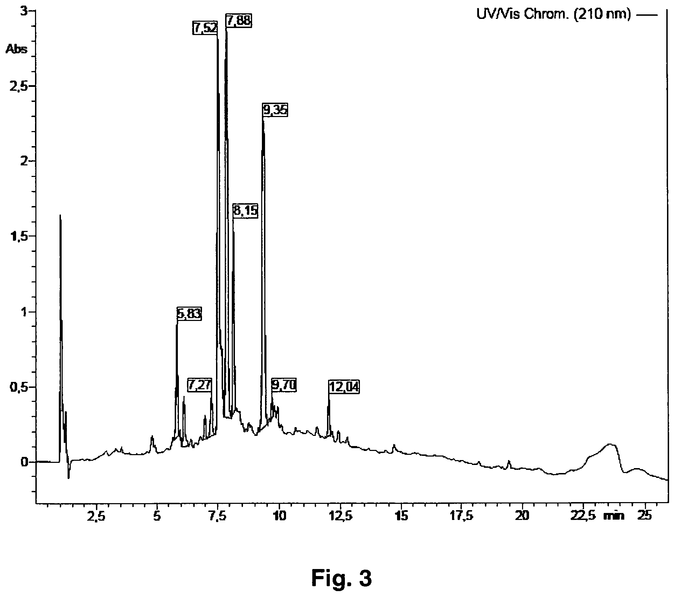

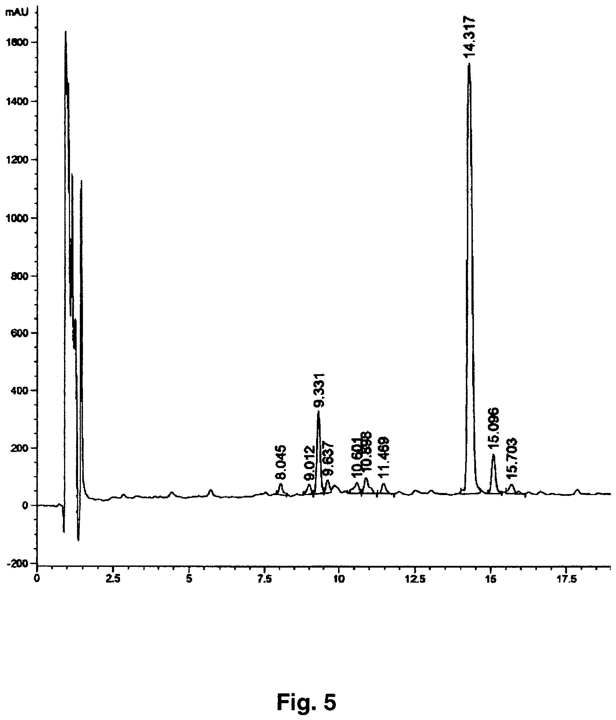

A) 3000 g of dried *Cynara scolymus* leaves (Alfred Galke GmbH, Gittelde/Harz, Germany) are extracted for 30 minutes at room temperature with 12,000 ml 70% Ethanol twice using ultrasonic irradiation. The resulting supernatant is separated from the remaining material and concentrated under reduced pressure. The remaining aqueous phase is adjusted with water to a final volume of 3,000 ml and is extracted twice with 3,000 ml n-heptane and subsequently twice with 3,000 ml ethyl acetate by liquid / liquid separation. The combined organic n-heptane phases are analysed for the presence of IMD-009047 and are discarded in case of absence of Cynaropicrin. The ethyl acetate phases are dried (Na₂SO₄), the solvents are evaporated under reduced pressure and the amount of the crude dry extract is determined. This process allows for an enrichment of the medium polar organic compounds out of the artichoke leaves. Typical yields are 81 g enriched extract out of the ethyl acetate phase starting with 3,000 g of *Cynara scolymus* leaves. This enriched extract contains about 20 % Cynaropicrin. A typical analytical HPLC profile of this extract is shown in figure 3.
B) An alternative extract was prepared using 100 g hackled areal parts of *Cynara scolymus* which were heated under reflux with 700 g of a mixture of 80% methanol/20% water (w/w) for 90 min. After cooling to room temperature the resulted mixture was filtered over a folded filter. The separated material was washed with an additional amount of 150 g of extraction solvent of the same constitution as above. The volume of the combined solutions was 900 ml. Approximately 10 ml of this solution was evaporated to dryness (80°C, normal pressure) and yielded in 180 mg raw material. A sample of this extract was analysed by HPLC-UV/VIS method (see general experimental methods, gradient: begin 10% eluent B to 20 min 40% eluent B, 25 min 90 % B). The chromatogram is presented in Fig 5. The signal with the retention time of 14.3 min refers to Cynaropicrin (detected by the use of a wavelength of 210 nm) (obtained using the HPLC methods described in the general experimental procedures).

### Example 5: preparation of Cynaropicrin

Two liquid chromatography steps are optimised for purification of Cynaropicrin. An aliquot of the enriched extract as prepared above, e.g. in this case 81 g, is separated on reverse phase material by low- medium pressure liquid chromatography on a Chromabond® (Trademark by Machery-Nagel GmbH & Co. KG, Düren Germany) P300-20 C18 column, 370 x 90 mm, (Macherey & Nagel). The elution scheme as outlined in the following table is used in a batch procedure (defined solvent mix at defined volume used):

| % H₂0 | % Acetonitril | % 2-Propanol | Volumen / Fraction |
|---|---|---|---|
| 80 | 20 | 0 | 1 x 2000 mL (Fraction 1) |
| 60 | 40 | 0 | 1 x 1000 mL (Fraction 2) |
| 60 | 40 | 0 | 4 x 250 mL (Fraction 3) |
| 40 | 60 | 0 | 4 x 250 mL (Fraction 4) |
| 40 | 60 | 0 | 2 x 500 mL (Fraction 4) |
| 0 | 100 | 0 | 1 x 1000 mL (Fraction 4) |
| 0 | 0 | 100 | 1 x 4000 mL (Fraction 4) |

Eluent batches are acidified by addition of TFA to a final concentration of 0.1%. The separation is performed with a pressure of 2-3 bar at room temperature. The resulting fractions are analysed by analytical HPLC-UV-ELSD. Fractions containing Cynaropicrin are combined. A typical yield of this first step of purification is 14 g, containing already about 70 % Cynaropicrin, starting from 81 g extract.

The second and final purification step is performed at room temperature on a preparative HPLC system (Gilson Abimed, Ratingen, Germany), comprising Gilson Unipoint software, 305 or 306 binary pump system, 204 fraction collector, 155 UV-Vis detector, 806 manometric module, and 811C dynamic mixer, using gradients and stationary phases as described below. The purification is performed on Chromabond or Nucleodur C18 columns (130 x 40 mmm, Macherey & Nagel) with a flow rate of 20 ml/min whereas the fractions are collected each minute using following gradient and solvents, which were acidified with 0.1 % TFA.:

| % H₂0 | % Acetonitril | Time [min] |
|---|---|---|
| 100 | 0 | 0 |
| 50 | 50 | 50 |
| 0 | 100 | 60 |
| 0 | 100 | 130 |
| 100 | 0 | 135 |
| 100 | 0 | 145 |

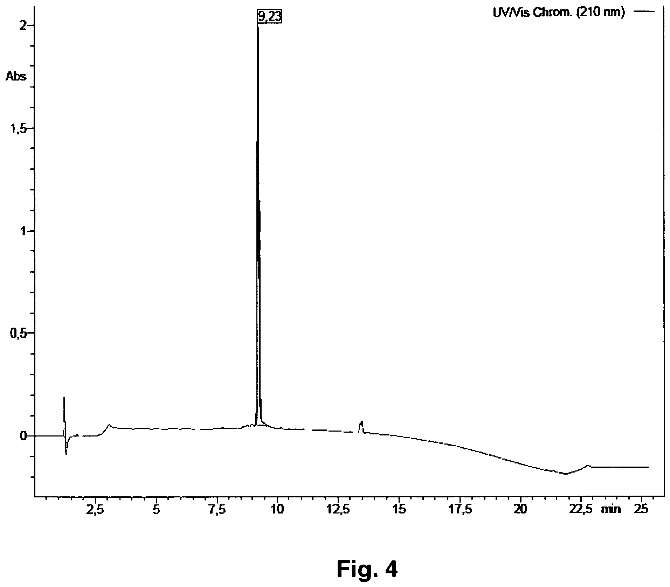

Using a UV/VIS-155 detector (Gilson, Langenfeld, Germany) the signals are detected at 210 nm and 254 nm. Cynaropicrin elutes typically in the range of 40 - 60 % acetonitrile. A typical yield of the procedure is 3.0 g cynaoropicrin starting with 4.6 g from the first purification step; this final product contains more than 90% cynaropicrin as shown in figure 4.

The final product of this example is used for the above described examples 1 to 3 for determining biological activities.

## Claims

1. A compound of the formula (I), for use in treating or preventing obesity in a warm-blooded animal 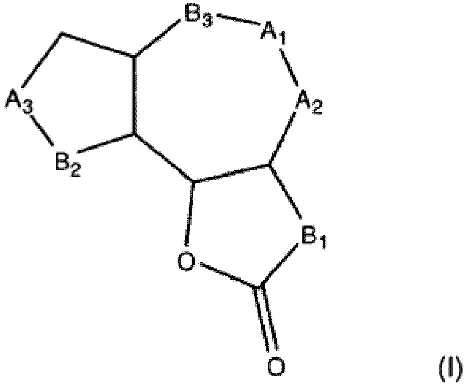 wherein
A₁, A₂ and A₃ independently from each other represent the diradicals **CH₂, **CH-O-R₁, or **C=O, and
B₁, B₂ and B₃ independently from each other represent the diradicals **C=CH₂, **CH-CH₂-R₁* or **C(OH)-CH₂-O-R₁;
in which R₁ is hydrogen, a straight-chain or branched-chain alkyl group having 1 to 12 carbon atoms, or a -C(O)-(CH₂)ₙ-CRₐ=CRₐR_{b} group (wherein n is zero or an integer from 1 to 12 and Rₐ and R_{b} independently from each other are selected from a group Z, consisting of hydrogen and a straight-chain or branched-chain alkyl group having 1 to 6 carbon atoms that is unsubstituted or substituted by hydroxyl), and R₁* is R₁ as defined above or a straight-chain or branched-chain alkoxy group having 1 to 12 carbon atoms,
where the compound of the formula (I) may be present in free form, in the form of a pharmaceutically and/or nutraceutically acceptable salt, in the form of tautomers and/or in the form of solvates.

2. A compound of the formula (I) according to Claim 1 for use in treating or preventing obesity in a warm-blooded animal wherein A₁ represents the diradical **CH₂ and B₃ represents the diradical **C=CH₂.

3. A compound of the formula (I), for use in treating or preventing obesity in a warm-blooded animal 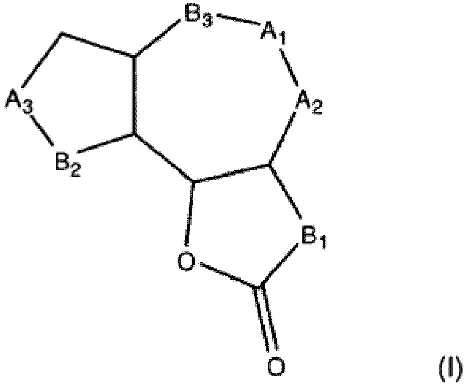 wherein
A₁ represents the diradical **CH₂;
A₂ and A₃ independently from each other represent the diradicals **CH₂, **CH-O-R₁, or **C=O,
B₃ represents the diradical **C=CH₂; and
B₁ and B₂ independently from each other represent the diradicals **C=CH₂, **CH-CH₂-R₁*, **C(OH)-CH₂-X or **C(OH)-CH₂-O-R₁;
in which X is selected from F, Cl and Br, R₁ is hydrogen, a straight-chain or branched-chain alkyl group having 1 to 12 carbon atoms, a cycloalkyl group having 3 to 10 carbon atoms and optionally comprising one or more ring heteroatoms, a carbohydrate having 5 to 12 carbon atoms, a substituted or unsubstituted aryl group with 6 to 12 carbon atoms which can comprise one or more ring heteroatoms, a substituted or unsubstituted C₆ to C₁₂ aryl-C₁₋₆ alkyl group which can comprise one or more heteroatoms, a substituted or unsubstituted aryloxy group where aryl has 6 to 12 ring atoms and can comprise one or more ring heteroatoms, a (-C(O)-Rₐ group, a (-C(S)-R^{a}) group, a (-C(O)-ORₐ) group, a (-(CH₂)ₙ-CRₐ=CRₐR_{b}) group, or a (-C(O)-(CH₂)ₙ-CRₐ=CRₐR_{b}) group (wherein n is zero or an integer from 1 to 12 and Rₐ and R_{b} independently from each other are selected from a group Z, consisting of hydrogen, a straight-chain or branched-chain alkyl group having 1 to 6 carbon atoms that is unsubstituted or substituted by hydroxyl, a cycloalkyl group having 3 to 10 carbon atoms and optionally comprising one or more ring heteroatoms, and a substituted or unsubstituted C₆ to C₁₂-aryl-(C₁ to C₆)-alkyl group which can comprise one or more heteroatoms), and R₁* is R₁ as defined above or a straight-chain or branched-chain alkoxy group having 1 to 12 carbon atoms, or **C(Rx)(Ry) where Rx, Ry and the binding carbon atom together form a cycloalkyl group having 3 to 10 carbon atoms and optionally comprising one or more ring heteroatoms, wherein if one or more heteroatoms are present they selected from S, N, NH and O
where the compound of the formula (I) is present in free form, in the form of a pharmaceutically and/or nutraceutically acceptable salt, in the form of tautomers and/or in the form of solvates; and
wherein the optional substituents of the aryl group with 6 to 12 carbon atoms which can comprise one or more ring heteroatoms, C₆ to C₁₂ aryl-C₁₋₆ alkyl group which can comprise one or more heteroatoms and aryloxy group where aryl has 6 to 12 ring atoms and can comprise one or more ring heteroatoms are selected from the group consisting of C₁-C₇alkyl, hydroxy, C₁-C₇-alkoxy, C₁-C₇alkanoyloxy, C₁-C₇alkoxycarbooxy, C₁-C₇alkanesulfonyloxy, phenyl-C₁-C₇-alkoxy, amino, N-mono- or N,N-di-(C₁-C₇-alkyl, C₁-C₇ alkanoyl, C₁-C₇-alkoxycarbonyl, C₁-C₇-alkanesulfonyl and/or phenyl-C₁-C₇-alkyl)-amino, carboxy, C₁-C₇-alkoxycarbonyl, carbamoyl, N-mono- or N,N-di-(C₁-C₇-alkyl)-carbamoyl, sulfamoyl, N-mono- or N,N-di-(C₁-C₇-alkyl)-sulfamoyl and cyano.

4. An extract of the plant or plant parts of a plant of a genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella for use in treating or preventing obesity in a warm-blooded animal, wherein said extract comprises a compound of formula (I) as defined in any of Claims 1-3 in free form, in the form of a pharmaceutically and/or nutraceutically acceptable salt, in the form of tautomers, and/or in the form of solvates.

5. A compound of formula (I) according to claim 3 for use in treating or preventing obesity in a warm-blooded animal selected from the group consisting of 3-Epi-11,13-dihydrodeacylcynaropicrin , Subexpinnatin, 11,13-Dihydrodeacylcynaropicrin, 11 beta, 13-Dihydrocynaropicrin, Isoamberboin, 3,11,13-Trihydroxy-10(14)-guaien-12,6-olide, Dehydrocyanaropicrin, Sibthorpin, 8-Deoxy-11,13-dihydroxygrosheimin, Isolipidiol , 8-Hydroxy-3-oxo-4(15),10(14)-guaiadien-12,6-olide, 3,8-Dihydroxy-10(14),11(13)-guaiadien-12,6-olide, Grossheimin, Integrifolin, 8beta-Hydroxydehydrozaluzanin C, Cynaropicrin, 13-Chloro-3,11-dihydroxy-4(15),10(14)-guaiadien-12,6-olide, 3-Acetyl-13-chloro-13-deoxysolstitialin, 8-Deoxy-11-hydroxy-13-chlorogrosheimin, Cynarascoloside A, Cynarascoloside B, Cynarascoloside C, Cynarinin A, and Cynarinin B, where the compound of the formula (I) may be present in free form, in the form of a pharmaceutically and/or nutraceutically acceptable salt, in the form of tautomers, and/or in the form of solvates.

6. An extract of the plant or plant parts of a plant of the genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella for use in treating or preventing obesity in a warm-blooded animal, comprising a compound of the formula (I) as defined in Claim 5 in free form, in the form of a pharmaceutically and/or nutraceutically acceptable salt, in the form of tautomers and/or in the form of solvates.

7. A compound of the formula (I) for use in treating or preventing obesity in a warm-blooded animal, where the compound of the formula (I) is Cynaropicrin, or a pharmaceutically and/or nutraceutically acceptable salt and/or solvate thereof.

8. An extract of the plant or plant parts of a plant of the genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella for use in treating or preventing obesity in a warm-blooded animal, wherein said extract comprises a compound of the formula (I) in free form, in the form of a pharmaceutically and/or nutraceutically acceptable salt, in the form of tautomers, and/or in the form of solvates, in which the compound of formula (I) is as defined in Claim 7.

9. The use of a compound of the formula (I) as defined in any one of claims 1, 2, 3, 5 or 7 for the manufacture of a pharmaceutical and/or nutraceutical preparation and/or dietary supplement and/or functional food for use in treating or preventing obesity in a warm-blooded animal, where the compound of the formula (I) may be in free form, in the form of a pharmaceutically and/or nutraceutically acceptable salt, in the form of tautomers, and/or in the form of solvates.

10. The use of a compound of the formula (I) as defined in any one of claims 4, 6 or 8 for the manufacture of a pharmaceutical and/or nutraceutical preparation and/or dietary supplement and/or functional food for use in treating or preventing obesity in a warm-blooded animal wherein the compound of formula (I) is in the form of an extract of the plant or plant parts of a plant of a genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella, each comprising a compound of the formula (I) in free form, in the form of a pharmaceutically and/or nutraceutically acceptable salt, in the form of tautomers and/or in the form of solvates.

11. The use according to claim 10 for the manufacture of a pharmaceutical preparation.

12. The use according to claim 10 for the manufacture of a nutraceutical preparation.

13. The use according to claim 10 for the manufacture of a dietary supplement.

14. The use according to claim 10 for the manufacture of or as a functional food.

15. A pharmaceutical and/or nutraceutical composition and/or dietary supplement and/or functional food for use in the therapeutic - including prophylactic - treatment of an animal and/or human for the regulation of body weight and/or fat loss and/or for the management of obesity, comprising a compound of the formula (I) as defined in any one of claims 1, 2, 3, 5 or 7 or an extract as defined in any one of claims 4, 6, or 8, where the compound of the formula (I) may be present in free form, in the form of a pharmaceutically and/or nutraceutically acceptable salt, in the form of tautomers, and/or in the form of solvates.

16. The non-therapeutic use of a compound of the formula (I) as defined in any one of claims 1, 2, 3, 5 or 7, for the regulation of body weight and/or body fat of a warm-blooded animal, comprising administering said compound to said animal, where the compound can also be used in the form of a cosmetically acceptable salt, in the form of tautomers, and/or in the form of solvates.

17. The non-therapeutic use according to Claim 16 of a compound of the formula (I) as defined in any one of claims 4, 6 or 8 in the form of an extract of the plant or plant parts of a plant of a genus selected from the group consisting of Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala and Volutarella, comprising a compound of the formula (I) in free form, in the form of a pharmaceutically and/or nutraceutically acceptable salt, in the form of tautomers and/or in the form of solvates.

18. A mixture of two or more compounds of formula (I) as defined in any one of claims 1 to 8 for use in treating or preventing obesity in a warm-blooded animal.

19. The non-therapeutic use of a mixture of two or more compounds of formula (I) as defined in any one of claims 1 to 8 for the regulation of body weight and/or body fat of a warm-blooded animal.

## Patentansprüche

1. Chemische Verbindung der Formel (I) zur Verwendung bei der Behandlung oder Vorbeugung von Fettleibigkeit bei einem Warmblüter 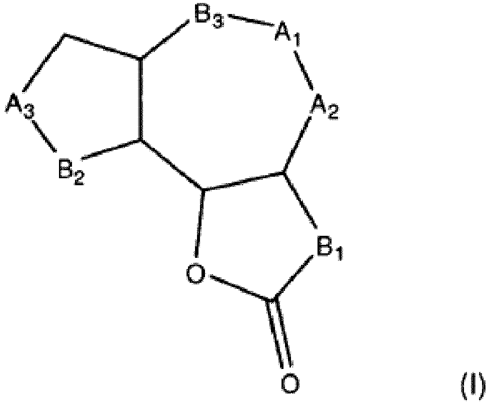 wobei
A₁, A₂ und A₃ unabhängig voneinander die Biradikale **CH₂, **CH-O-R₁ oder **C=O bedeuten und
B₁, B₂ und B₃ unabhängig voneinander die Biradikale **C=CH₂, **CH-CH₂-R₁* oder **C(OH)-CH₂-O-R₁ bedeuten;
wobei R₁ Wasserstoff, eine geradkettige oder verzweigtkettige Alkylgruppe mit 1 bis 12 Kohlenstoffatomen oder eine -C(O)-(CH₂)ₙ-CRₐ=CRₐR_{b}-Gruppe ist (wobei n gleich Null oder eine ganze Zahl von 1 bis 12 ist und Rₐ und R_{b} unabhängig voneinander aus einer Gruppe Z ausgewählt sind, die aus Wasserstoff und einer geradkettigen oder verzweigtkettigen Alkylgruppe mit 1 bis 6 Kohlenstoffatomen besteht und durch Hydroxyl unsubstituiert oder substituiert ist) und Rᵢ* gemäß der obigen Definition gleich R₁ ist oder eine geradkettige oder verzweigtkettige Alkoxy-Gruppe mit 1 bis 12 Kohlenstoffatomen,
wobei die chemische Verbindung der Formel (I) in freier Form, in Form eines pharmazeutischen und/oder nutrazeutischen Salzes, in Form von Tautomeren und/oder in Form von Solventraffinaten vorliegen kann.

2. Chemische Verbindung der Formel (I) gemäß Anspruch 1 zur Verwendung bei der Behandlung oder Vorbeugung von Fettleibigkeit bei einem Warmblüter, wobei A₁ für das Biradikal **CH₂ und B₃ für das Biradikal **C=CH₂ steht.

3. Chemische Verbindung der Formel (I) zur Verwendung bei der Behandlung oder Vorbeugung von Fettleibigkeit bei einem Warmblüter 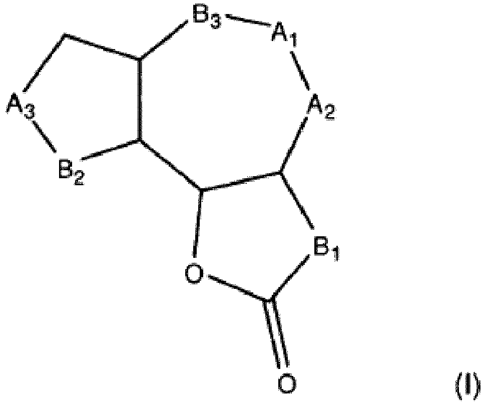 wobei
A₁ für das Biradikal **CH₂ steht;
A₂ und A₃ unabhängig voneinander die Biradikale **CH₂, **CH-O-R₁ oder **C=O bedeuten,
B₃ für das Biradikal **C=CH₂ steht und;
B₁ und B₂ unabhängig voneinander die Biradikale **C=CH₂, **CH-CH₂-R₁*, **C(OH)-CH₂-X oder **C(OH)-CH₂-O-R₁ bedeuten;
wobei X aus F, Cl und Br ausgewählt ist, R₁ Wasserstoff, eine geradkettige oder verzweigtkettige Alkoxy-Gruppe mit 1 bis 12 Kohlenstoffatomen ist, eine Cycloalkylgruppe mit 3 bis 10 Kohlenstoffatomen und gegebenenfalls mit einem Ring-Heteroatom oder mehreren Ring-Heteroatomen ist, ein Kohlenhydrat mit 5 bis 12 Kohlenstoffatomen ist, eine substituierte oder unsubstituierte Arylgruppe mit 6 bis 12 Kohlenstoffatomen ist, die ein Ring-Heteroatom oder mehrere Ring-Heteroatome, eine substituierte oder unsubstituierte C₆ bis C₁₂ Aryl-C₁₋₆-Alkylgruppe umfassen kann, die ein Heteroatom oder mehrere Heteroatome, eine substituierte oder unsubstituierte Phenolatgruppe umfasst, wobei Aryl 6 bis 12 Ringatome aufweist und ein Ring-Heteroatom oder mehrere Ring-Heteroatome, eine (-C(O)-Rₐ)-Gruppe, eine (-C(S)-Rₐ)-Gruppe, eine (-C(O)-ORₐ)-Gruppe, eine (-(CH₂)ₙ-CRₐ=CRₐR_{b})-Gruppe oder eine (-C(O)-(CH₂)ₙ-CRₐ=CRₐR_{b})-Gruppe umfassen kann (wobei n gleich Null oder eine ganze Zahl von 1 bis 12 ist und Rₐ und R_{b} unabhängig voneinander aus einer Gruppe Z ausgewählt sind, die aus Wasserstoff, einer geradkettigen oder verzweigtkettigen Alkylgruppe mit 1 bis 6 Kohlenstoffatomen besteht und durch Hydroxyl unsubstituiert oder substituiert ist, einer Cycloalkylgruppe mit 3 bis 10 Kohlenstoffatomen und gegebenenfalls ein Heteroatom oder mehrere Ring-Heteroatome und eine substituierte oder unsubstituierte C₆ bis C₁₂-Aryl-(C₁ bis C₆)-Alkylgruppe aufweist, die ein Heteroatom oder mehrere Heteroatome enthalten kann) und R₁* gemäß der obigen Definition gleich R₁ ist oder eine geradkettige oder verzweigtkettige Alkoxy-Gruppe mit 1 bis 12 Kohlenstoffatomen oder **C(Rx)(Ry) ist, wobei Rx, Ry und das Bindungskohlenstoffatom zusammen eine Cycloalkylgruppe mit 3 bis 10 Kohlenstoffatomen und gegebenenfalls ein Heteroatom oder mehrere Ring-Heteroatomen bilden, wobei diese aus S, N, NH und O ausgewählt sind, wenn ein Heteroatom oder mehrere Heteroatome vorhanden sind;
wobei die chemische Verbindung der Formel (I) in freier Form, in Form eines pharmazeutischen und/oder nutrazeutischen Salzes, in Form von Tautomeren und/oder in Form von Solventraffinaten vorliegt; und
wobei die optionalen Substituenten der Arylgruppe mit 6 bis 12 Kohlenstoffatomen, die ein oder mehrere Ring-Heteroatome umfassen können, die C₆ bis C₁₂ Aryl-C₁₋₆-Alkylgruppe, die ein oder mehrere Heteroatome umfassen kann und die Phenolatgruppe, wobei Aryl 6 bis 12 Ringatome aufweist und ein oder mehrere Ring-Heteroatome umfassen kann, aus der Gruppe bestehend aus C₁-C₇Alkyl, Hydroxyl, C₁-C₇-Alkoxyl, C₁-C₇Alkanoyloxyl, C₁-C₇Alkoxylcarboxyl, C₁-C₇Alkanosulfonyloxyl, Phenyl-C₁-C₇-Alkoxyl, Amino-, N-mono- oder N,N-di-(C₁-C₇-Alkyl, C₁-C7 Alkanoyl, C₁-C₇-Alkoxylcarbonyl, C₁-C₇-Alkanosulfonyl und/oder Phenyl-C₁-C₇-Alkyl)-Amino, Carboxyl, C₁-C₇-Alkoxylcarbonyl, Carbamoyl, N-mono- oder N,N-di-(C₁-C₇-Alkyl)-Carbamoyl, Sulfamoyl, N-mono- oder N,N-di-(Ci-C₇-Alkyl)-Sulfamoyl und Cyano ausgewählt sind.

4. Ein Pflanzenextrakt aus einer Pflanzengruppe der Gattungen Cynara, Centaurea, Saussurea, Amberboa, Grossheimia, Tricholepsis, Cheirolophus, Makroclinidium, Vernonia, Ixeris, Jurinea, Ainsliaea, Pseudostifftia, Crepis, Cartolepsis, Andryala und Volutarella zur Verwendung bei der Behandlung oder Verhinderung von Fettleibigkeit in einem Warmblüter, wobei das Extrakt eine Verbindung der Formel (I) entsprechend den Ansprüchen 1 bis 3 in freier Form, in Form eines pharmazeutisch und/oder nutrazeutisch duldbaren Salzes, in Form von Tautomeren und/oder in Form von Solventraffinaten ist.

5. Chemische Verbindung der Formel (I) gemäß Anspruch 3 zur Verwendung bei der Behandlung oder Verhinderung von Fettleibigkeit in einem Warmblüter, die aus der Gruppe bestehend aus 3-Epi-11,13- Dihydrodeacylcynaropicrin, Subexpinnatin, 11,13-Dihydrodeacylcynaropicrin, 11 beta, 13-Dihydrocynaropicrin, Isoamberboin, 3,11,13-Trihydroxyl-10(14)-Guaien-12,6-olide, Dehydrocyanaropicrin, Sibthorpin, 8-Deoxyl-11,13-Dihydroxygrosheimin, Isolipidiol, 8-Hydroxyl-3-Oxo-4(15),10(14)-Guaiadien-12,6-Olide, 3,8-Dihydroxyl-10(14),11(13)-Guaiadien-12,6-Olide, Grossheimin, Integrifolin, 8beta-Hydroxydehydrozaluzanin C, Cynaropicrin, 13-Chloro-3,11-Dihydroxyl-4(15),10(14)-Guaiadien-12,6-Olide, 3-Acetyl-13-Chloro-13-Deoxysolstitialin, 8-Deoxyl-11-Hydroxyl-13-Chlorogrosheimin, Cynarascoloside A, Cynarascoloside B, Cynarascoloside C, Cynarinin A und Cynarinin B ausgewählt ist, wobei die chemische Verbindung der Formel (I) in freier Form, in Form eines pharmazeutischen und/oder nutrazeutischen Salzes, in Form von Tautomeren und/oder in Form von Solventraffinaten vorliegen kann.

6. Ein Pflanzenextrakt aus einer Pflanze der Gattungen Cynara, Centaurea, Saussurea, Amberboa, Grossheimia, Tricholepsis, Cheirolophus, Makroclinidium, Vernonia, Ixeris, Jurinea, Ainsliaea, Pseudostifftia, Crepis, Cartolepsis, Andryala und Volutarella zur Verwendung bei der Behandlung oder Verhinderung von Fettleibigkeit in einem Warmblüter, das eine Verbindung der Formel (I) entsprechend dem Anspruch 5 in freier Form, in Form eines pharmazeutisch und/oder nutrazeutisch duldbaren Salzes, in Form von Tautomeren und/oder in Form von Solventraffinaten umfasst.

7. Chemische Verbindung der Formel (I) zur Verwendung bei der Behandlung oder Vorbeugung von Fettleibigkeit bei einem Warmblüter, wobei die chemische Verbindung der Formel (I) Cynaropicrin oder dessen pharmazeutisch und/oder nutrazeutisch duldbares Salz und/oder Solventraffinat ist.

8. Ein Pflanzenextrakt aus einer Pflanze der Gattungen Cynara, Centaurea, Saussurea, Amberboa, Grossheimia, Tricholepsis, Cheirolophus, Makroclinidium, Vernonia, Ixeris, Jurinea, Ainsliaea, Pseudostifftia, Crepis, Cartolepsis, Andryala und Volutarella zur Verwendung bei der Behandlung oder Verhinderung von Fettleibigkeit in einem Warmblüter, wobei das Extrakt eine Verbindung der Formel (I) in freier Form, in Form eines pharmazeutisch und/oder nutrazeutisch duldbaren Salzes, in Form von Tautomeren und/oder in Form von Solventraffinaten umfasst, in der die chemische Verbindung der Formel (I) in Anspruch 7 entspricht.

9. Verwendung einer chemischen Verbindung der Formel (I) gemäß einem der Ansprüche 1, 2, 3, 5 oder 7 zur Herstellung eines pharmazeutischen und/oder nutrazeutischen Präparats und/oder Nahrungsergänzungsmittels und/oder funktionellen Nahrungsmittels zur Verwendung bei der Behandlung oder Verhinderung von Fettleibigkeit bei einem Warmblüter, wobei die chemische Verbindung der Formel (I) in freier Form, in Form eines pharmazeutischen und/oder nutrazeutischen Salzes, in Form von Tautomeren und/oder in Form von Solventraffinaten vorliegen kann.

10. Verwendung einer chemischen Verbindung der Formel (I) gemäß einem der Ansprüche 4, 6 oder 8 zur Herstellung eines pharmazeutischen und/oder nutrazeutischen Präparats und/oder Nahrungsergänzungsmittels und/oder funktionellen Nahrungsmittels zur Verwendung bei der Behandlung oder Verhinderung von Fettleibigkeit in einem Warmblüter, wobei die chemische Verbindung der Formel (I) aus einem Pflanzenextrakt aus einer Pflanze der Gattungen Cynara, Centaurea, Saussurea, Amberboa, Grossheimia, Tricholepsis, Cheirolophus, Makroclinidium, Vernonia, Ixeris, Jurinea, Ainsliaea, Pseudostifftia, Crepis, Cartolepsis, Andryala und Volutarella besteht, welches jeweils eine chemische Verbindung der Formel (I) in freier Form, in Form eines pharmazeutisch und/oder nutrazeutisch duldbaren Salzes, in Form von Tautomeren und/oder in Form von Solventraffinaten umfasst.

11. Verwendung gemäß Anspruch 10 zur Herstellung eines Arzneimittels.

12. Verwendung gemäß Anspruch 10 zur Herstellung eines nutrazeutischen Präparats.

13. Verwendung gemäß Anspruch 10 zur Herstellung eines Nahrungsergänzungsmittels.

14. Verwendung gemäß Anspruch 10 zur Herstellung eines funktionellen Nahrungsmittels.

15. Pharmazeutische(s) und/oder nutrazeutische(s) Zusammensetzung und/oder Nahrungsergänzungsmittel und/oder funktionelles Nahrungsmittel zur Verwendung in der therapeutischen - einschließlich prophylaktischen - Behandlung eines Tieres und/oder Menschen zur Regulierung des Körpergewichts und/oder des Fettabbaus und/oder der Behandlung von Fettleibigkeit, umfassend eine chemische Verbindung der Formel (I) gemäß einem der Ansprüche 1, 2, 3, 5 oder 7 oder ein Extrakt gemäß einem der Ansprüche 4, 6 oder 8, wobei die chemische Verbindung der Formel (I) in freier Form, in Form eines pharmazeutischen und/oder nutrazeutischen Salzes, in Form von Tautomeren und/oder in Form von Solventraffinaten vorliegen kann.

16. Nicht-therapeutische Verwendung einer chemischen Verbindung der Formel (I) gemäß einem der Ansprüche 1, 2, 3, 5 oder 7 zur Regulierung des Körpergewichts und/oder des Körperfetts eines Warmblüters, umfassend die Verabreichung der chemischen Verbindung an ein Tier, wobei die chemische Verbindung auch in Form eines kosmetisch duldbaren Salzes in Form von Tautomeren und/oder in Form von Solventraffinaten verwendet werden kann.

17. Nicht-therapeutische Verwendung gemäß Anspruch 16 einer chemischen Verbindung der Formel (I) gemäß einem der Ansprüche 4, 6 oder 8 als Pflanzenextrakt aus einer Pflanze der Gattungen Cynara, Centaurea, Saussurea, Amberboa, Grossheimia, Tricholepsis, Cheirolophus, Makroclinidium, Vernonia, Ixeris, Jurinea, Ainsliaea, Pseudostifftia, Crepis, Cartolepsis, Andryala und Volutarella, welches jeweils eine chemische Verbindung der Formel (I) in freier Form, in Form eines pharmazeutisch und/oder nutrazeutisch duldbaren Salzes, in Form von Tautomeren und/oder in Form von Solventraffinaten umfasst.

18. Mischung aus zwei oder mehr chemischen Verbindungen der Formel (I) gemäß einem der Ansprüche 1 bis 8 zur Verwendung bei der Behandlung oder Vorbeugung von Fettleibigkeit bei einem Warmblüter.

19. Nicht-therapeutische Verwendung einer Mischung aus zwei oder mehr chemischen Verbindungen der Formel (I) gemäß einem der Ansprüche 1 bis 8 zur Regulierung des Körpergewichts und/oder des Körperfetts eines Warmblüters.

## Revendications

1. Un composé de la formule (I) à utiliser pour traiter ou prévenir l'obésité chez un animal à sang chaud 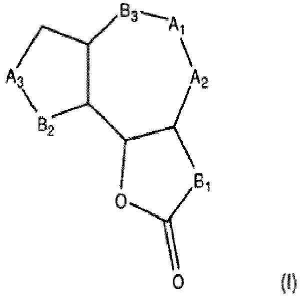 dans lequel
A1, A2 et A3 représentent chacun indépendamment les diradicaux **CH2, **CH-O-R1, ou **C=O, et
B1, B2, et B3 représentent chacun indépendamment les diradicaux **C=CH2, **CH-CH2-R1* ou **C(OH)-CH2-O-R1 ;
dans lequel R1 est hydrogène, un groupe alkyle à chaîne droite ou ramifiée ayant 1 à 12 atomes de carbone, ou un groupe -C(O)-(CH2)n-CRaRb (dans lequel n est zéro ou entier de 1 à 12 et Ra et Rb sont chacun indépendamment sélectionnés d'un groupe Z, composé d'hydrogène et d'un groupe alkyle ayant 1 à 6 atomes de carbone qui est non substitué ou substitué par l'hydroxyle), et R1* est R1 tel que défini ci-dessus ou un groupe alkyle à chaîne droite ou ramifiée ayant 1 à 12 atomes de carbone,
où le composé de la formule (I) peut être présent dans une forme libre, dans une forme d'un sel pharmaceutiquement et / ou nutraceutiquement acceptable, dans la forme de tautomères et / ou dans la forme de solvates.

2. Un composé de la formule (I) selon la revendication 1 à utiliser pour traiter ou prévenir l'obésité chez un animal à sang chaud dans lequel A1 représente le diradical **CH2 et B3 représente le diradical **C=CH2.

3. Un composé de la formule (I) selon la revendication 1 à utiliser pour traiter ou prévenir l'obésité chez un animal à sang chaud. 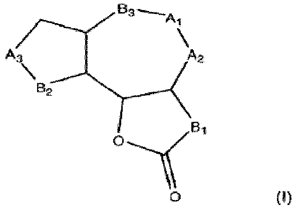 dans lequel
A1 représente le diradical **CH2 ;
A2 et A3 représentent chacun indépendamment les diradicaux **CH2, **CH-O-R1, ou **C=O,
B3 représente le diradical **C=CH2 ; et
B1 et B2 représentent chacun indépendamment les diradicaux **C=CH2, **CH-CH2-R1*, **C(OH)-CH2-X ou **C(OH)-CH2-O-R1 ;
dans lequel X est sélectionné à partir de F, Cl et Br, R1 est l'hydrogène, un groupe alkyle à chaîne droite ou ramifiée ayant 1 à 12 atomes de carbone, un groupe cycloalkyle ayant 3 à 10 atomes de carbone et comprenant facultativement un ou plusieurs hétéroatomes, un carbohydrate ayant 5 à 12 atomes de carbone, un groupe aryle substitué ou non substitué avec 6 à 12 atomes de carbone qui peuvent comprendre un ou plusieurs hétéroatomes cycliques, un groupe alkyle C6 à C12 aryle-C1-6 pouvant comprendre un ou plusieurs hétéroatomes, un groupe aryloxy substitué ou non substitué où aryle a 6 à 12 atomes cycliques et peut comprendre un ou plusieurs hétéroatomes cycliques, un groupe (-C(O)-Ra, un groupe (-C(S)-R^{a}), un groupe (-C(O)-ORa), un groupe (-(CH2)n-CRa=CRaRb), ou un groupe (-C(O)-(CH2)n-CRa=CRaRb) (dans lequel n est zéro ou un entier de 1 à 12 et Ra et Rb sont chacun indépendamment sélectionnés à partir d'un groupe Z, composé d'hydrogène, un groupe alkyle à chaîne droite ou ramifiée ayant 1 à 6 atomes de carbone qui est non substitué ou substitué by l'hydroxyle, un groupe cycloalkyle ayant 3 à 10 atomes de carbone et comprenant facultativement un ou plusieurs hétéroatomes cycliques, et un groupe alkyle C6 à C12-aryle-(C1 à C6) substitué ou non substitué pouvant comprendre un ou plusieurs hétéroatomes), et R1* est R1 tel que défini ci-dessus ou un groupe alcoxyle à chaîne droite ou ramifiée ayant 1 à 12 atomes de carbone, ou **C(Rx)(Ry) où Rx, Ry et l'atome de carbone de liaison forment ensemble un groupe cycloalkyle ayant 3 à 10 atomes de carbone et comprenant facultativement un ou plusieurs hétéroatomes cycliques, dans lequel si un ou plusieurs hétéroatomes sont présents, ils sont sélectionnés de S, N, NH et O où le composé de la formule (I) est présent dans une forme libre, dans la forme d'un sel pharmaceutiquement et / ou nutraceutiquement acceptable, dans la forme de tautomères et / ou dans la forme de solvates ; et
dans lequel les substituants facultatifs du groupe aryle avec 6 à 12 atomes de carbone pouvant comprendre un ou plusieurs hétéroatomes, un groupe alkyle C6 à C12 aryle-C1-6 qui peut comprendre un ou plusieurs hétéroatomes et un groupe aryloxy où aryle a 6 à 12 atomes cycliques et peut comprendre un ou plusieurs hétéroatomes cycliques sont sélectionnés d'un groupe composé d'alkyle C1-C7, d'hydroxy, d'alkoxy C1-C7, d'alkanoyloxy C1-C7, d'alkoxycarbooxy C1-C7, d'alkanesulfonyloxy C1-C7, de phényle-C1-C7-alkoxy, d'aminé, de N-mono-N, ou de N-di-(C1-C7-alkyle, C1-C7 alkanoyl, C1-C7-alkoxycarbonyl, C1-C7-alkanesulfonyle et / ou de phényle-C1-C7-alkyle)-de carbamoyle, sulfamoyle, de N-mono- ou de N, N-di-(C1-C7-alkyle)-sulfamoyle et de cyano.

4. Un extrait de la plante ou des parties de la plante d'une plante d'un genre sélectionné d'un groupe composé de Cynara, Centaurea, Saussurea, Amberboa, Grossheimia, Tricholepsi, Cheiroplophus, Macroclinifiul, Vernonia, Ixeris, Jurinea, Ainsliaea, Pseudostifftia, Crepis, Cartolepsis, Andryala et Volutarella à utiliser pour traiter ou prévenir l'obésité chez un animal de sang chaud, dans lequel ledit extrait comprend un composé de la formule (I) tel que défini dans les revendications 1-3 dans la forme libre, dans la forme d'un sel pharmaceutiquement et / ou nutraceutiquement acceptable, dans la forme de tautomères, et / ou dans la forme de solvates.

5. Un composé d'une formule (I) selon la revendication 3 à utiliser pour traiter ou prévenir l'obésité chez un animal à sang chaud sélectionné du groupe composé de 3-Epi-11, 13-dihydrodéacylcynaropicrine, subexpinnatin, 11, 13-dihydrodéacyclycynaropicrine, 11 béta, 13-dihydrocynaropicrine, isoamberboin, 3, 11, 13-trihydroxy-10 (14)-guaien-12, 6-olide, déhydrocyanaropicrine, sibthorpine, 8-déoxy-11, 13-dihydroxygrosheimin, Isolipidiol, 8-Hydroxy-3-oxo-4(15),10(14)-guaiadien-12,6-olide, 3,8-Dihydroxy-10(14),11(13)-guaiadien-12,6-olide, Grossheimin, Intégrifolié, 8 béta-Hydroxydehydrozaluzanin C, Cynaropicrine, 13-Chloro-3,11-dihydroxy-4(15),10(14)-guaiadien-12,6-olide, 3-Acetyle-13-chloro-13-deoxysolstitialin, 8-Déoxy-11-hydroxy-13-chlorogrosheimine, Cynarascoloside A, Cynarascoloside B, Cynarascoloside C, Cynarinine A, et Cynarinine B, où le composé de la formule (I) peut être présent dans la forme libre, dans la forme d'un sel pharmaceutiquement et / ou nutraceutiquement acceptable, dans la forme de tautomères, et / ou dans la forme de solvates.

6. Un extrait de la plante ou des parties de la plante d'une plante du genre sélectionné du groupe composé de Cynara, Centaurea, Saussurea, Amberboa, Grossheimia, Tricholepsis,Cheirolophus, Macroclinidium, Vernonia, Ixeris, Jurinea, Ainsliaea, Pseudostifftia, Crepis, Cartolepsis, Andryala et Volutarella à utiliser pour traiter ou prévenir l'obésité chez un animal à sang chaud, comprenant un composé de la formule (I) tel que défini dans la revendication 5 dans la forme libre, dans la forme d'un sel pharmaceutiquement et / ou nutraceutiquement acceptable, dans la forme de tautomères et / ou dans la forme de solvates.

7. Un composé de la formule (I) à utiliser pour traiter ou prévenir l'obésité chez un animal à sang chaud, où le composé de la formule (I) est Cynaropicrine, ou un sel pharmaceutiquement et / ou nutraceutiquement acceptable et / ou un solvate de celui-ci.

8. Un extrait de la plante ou des parties de la plante d'une plante du genre sélectionné du groupe composé de Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala et Volutarella à utiliser pour traiter ou prévenir l'obésité chez un animal à sang chaud, dans lequel ledit extrait comprend un composé de la formule (I) dans la forme libre, dans la forme d'un sel pharmaceutiquement et / ou nutraceutiquement acceptable, dans la forme de tautomères, et / ou dans la forme de solvates, dans lequel le composé de la formule (I) est tel que défini dans la revendication 7.

9. L'utilisation d'un composé de la formule (I) tel que défini dans l'une des revendications 1, 2, 3, 5 ou 7 pour la fabrication d'une préparation pharmaceutique et / ou nutraceutique et / ou un aliment fonctionnel à utiliser pour traiter ou prévenir l'obésité chez l'animal à sang chaud, où le composé de la formule (I) peut être dans la forme libre, dans la forme d'un sel pharmaceutiquement et / ou nutraceutiquement acceptable, dans la forme de tautomères, et / ou dans la forme de solvates.

10. L'utilisation d'un composé de la formule (I) tel que défini dans l'une des revendications 4, 6 ou 8 pour la fabrication d'une préparation pharmaceutique et / ou nutraceutique et / ou un complément alimentaire et / ou un aliment fonctionnel à utiliser pour traiter et ou prévenir l'obésité chez un animal à sang chaud dans lequel le composé de la formule (I) est dans la forme d'un extrait de la plante ou des parties de la plante d'une plante du genre sélectionné du groupe composé de Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala et Volutarella, chacun comprenant un composé de la formule (I) dans la forme libre, dans la forme d'un sel pharmaceutiquement et / ou nutraceutiquement acceptable, dans la forme de tautomères et / ou dans la forme de solvates.

11. L'utilisation selon la revendication 10 pour la fabrication d'une préparation pharmaceutique.

12. L'utilisation selon la revendication 10 pour la fabrication d'une préparation nutraceutique.

13. L'utilisation selon la revendication 10 pour la fabrication d'un complément alimentaire.

14. L'utilisation selon la revendication 10 pour la fabrication de ou en tant qu'aliment fonctionnel.

15. Une composition pharmaceutique ou nutraceutique et / ou un complément alimentaire et / ou un aliment fonctionnel à utiliser dans le traitement thérapeutique - y compris prophylactique - d'un animal et / ou un humain pour la régulation du poids corporel et / ou la perte de graisse et / ou pour la gestion de l'obésité, comprenant un composé de la formule (I) tel que défini dans l'une des revendications 1, 2, 3, 5 ou 7 ou un extrait tel que défini dans l'une des revendications 4, 6, ou 8, où le composé de la formule (I) peut être présent dans la forme libre, dans la forme d'un sel pharmaceutiquement et / ou nutraceutiquement acceptable, dans la forme de tautomères, et / ou dans la forme de solvates.

16. L'utilisation non thérapeutique d'un composé de la formule (I) tel que défini dans l'une des revendications 1, 2, 3, 5 ou 7, pour la régulation du poids corporel et / ou la graisse corporelle d'un animal à sang chaud, comprenant l'administration dudit composé audit animal, où le composé peut également être utilisé dans la forme d'un sel cosmétiquement acceptable, dans la forme de tautomères, et / ou dans la forme de solvates.

17. L'utilisation non thérapeutique selon la revendication 16 d'un composé de la formule (I) tel que défini dans l'une des revendications 4, 6 ou 8 dans la forme d'un extrait de la plante ou des parties de la plante d'une plante d'un genre sélectionné du groupe composé de Cynara, Centaurea, Saussurea, Amberboa, Grossheimia, Tricholepsis, Cheirolophus, Macroclinidium, Vernonia, Ixeris, Jurinea, Ainsliaea, Pseudostifftia, Crepis, Cartolepsis, Andryala et Volutarella, comprenant un composé de la formule (I) dans la forme libre, dans la forme d'un sel pharmaceutiquement et / ou nutraceutiquement acceptable, dans la forme de tautomères et / ou dans la forme de solvates.

18. Un mélange de deux ou plusieurs composés de la formule (I) tel que défini dans l'une des revendications 1 à 8 à utiliser pour traiter ou prévenir l'obésité chez l'animal à sang chaud.

19. L'utilisation non thérapeutique d'un mélange de deux ou plusieurs composés de la formule (I) tel que défini dans l'une des revendications 1 à 8 pour la régulation du poids corporel et / ou la graisse corporelle d'un animal à sang chaud.
